# NEUE EUROPÄISCHE PATENTSCHRIFT

(11) **EP 3 319 569 B2**
(45) Veröffentlichungstag und Bekanntmachung des Hinweises auf die Entscheidung über den Einspruch: **12.08.2026**
(45) Hinweis auf die Patenterteilung: 19.01.2022
(21) Anmeldenummer: 16739063.2
(22) Anmeldetag: 06.07.2016
(51) Int. Cl.: A61F 9/008

(54) **OPHTHALMOLOGISCHE THERAPIEANORDNUNG ZUR BEARBEITUNG EINER FLÄCHE IN EINEM BEARBEITUNGSVOLUMEN EINES TRANSPARENTEN MATERIALS MITTELS EINER FOKUSSIERTEN STRAHLUNG**
OPHTHALMOLOGIC THERAPY ASSEMBLY FOR PROCESSING A SURFACE IN A PROCESSING VOLUME OF A TRANSPARENT MATERIAL BY MEANS OF FOCUSED RADIATION
DISPOSITIF THÉRAPEUTIQUE OPHTALMOLOGIQUE POUR L'USINAGE D'UNE SURFACE DANS UN VOLUME D'USINAGE D'UN MATÉRIAU TRANSPARENT AU MOYEN D'UN FAISCEAU FOCALISÉ

(30) Priorität: 09.07.2015 DE 102015212877
(43) Veröffentlichungstag der Anmeldung: 16.05.2018
(73) Patentinhaber: Carl Zeiss Meditec AG, 07745 Jena (DE)
(72) Erfinder: BERGT, Michael, 99425 Weimar (DE); HAMANN, Thomas, 07743 Jena (DE); POMRAENKE, Robert, 07743 Jena (DE)
(74) Vertreter: Rößner, Ulrike
(86) Internationale Anmeldenummer: PCT/EP2016/066033
(87) Internationale Veröffentlichungsnummer: WO 2017/005815

(56) Entgegenhaltungen:
- EP-A1- 2 412 341
- EP-A1- 2 412 342
- EP-A1- 2 596 774
- WO-A1-2016/050779
- DE-A1- 102011 085 046

## Beschreibung

Die vorliegende Erfindung betrifft eine Anordnung zur Bearbeitung einer Fläche in einem Bearbeitungsvolumen eines transparenten Materials mittels einer fokussierten Strahlung, die eine Einrichtung zur Erzeugung einer Strahlung, eine Optik zur Fokussierung der Strahlung in einem Fokus im Bearbeitungsvolumen, eine Einrichtung zur Veränderung der Lage des Fokus im Bearbeitungsvolumen und eine Steuereinrichtung, eingerichtet zur Steuerung der Anordnung, enthält. Die vorliegende Erfindung betrifft weiterhin ein Steuerprogrammprodukt und eine Planungseinheit für eine Anordnung zur Bearbeitung einer Fläche in einem Bearbeitungsvolumen eines transparenten Materials mittels einer fokussierten Strahlung.

Eine solche Anordnung wird vorzugsweise in der Augenmedizin als ophthalmologische Therapieanordnung eingesetzt. Das zu bearbeitende transparente Material kann dabei ein natürliches Augengewebe, beispielsweise die Linse, die Cornea oder der Kapselsack, oder auch ein künstliches Augenmaterial, beispielsweise eine künstliche Linse, sein.

Bislang üblich sind hierbei sogenannte Spiralscans. Diese sind beispielsweise in den Druckschritten US 5,984,916, DE 10 2011 085 046 A1 und DE 10 2011 085 047 A1 beschrieben, ein Ausführungsbeispiel ist in der Fig. 1a dargestellt. Ein Laserstrahl 1 wird mittels einer Optik 2, 201 in einem transparenten Material 3 fokussiert. Der Fokus 4 wird dabei durch ein Scansystem entlang einer Scanlinie 5, die hier aufgrund einer lateralen beliebig geschlossenen Bewegung, beispielsweise einer Rotationsbewegung, und einer langsamen z-Bewegung des Scansystems spiralförmig ist, durch ein transparentes Augengewebe 3 bewegt. Die Optik 2, 201 bestimmt dabei die Form des Fokuskegels 6. Um einen solchen Spiralscan zur Bearbeitung des Augengewebes 3 anwenden zu können, muss der durch die Optik 2, 201 erreichbare laterale Bereich für die Fokussierung des Lasers 1 groß genug sein, um alle Bearbeitungsgebiete ohne langsame laterale Bewegung der Optik 2, 201 zu erreichen. Die Optik 2, 201 ist dementsprechend groß und teuer. Aufgrund der erforderlichen Größe eignet sich diese Methode zudem auch nur bedingt für den Einsatz in flexiblen, beweglichen Geräten mit einem Schwenkarm.

Auch sind Schnitte mit einem Femtosekunden-Laser bekannt, die in vertikaler Richtung durch schnelle Scanbewegungen ausgeführt werden. Dies wird vertikales z-Wobbeln genannt und ist beispielsweise in der Druckschrift WO 2013/057318 A1 beschrieben. Die Fig. 1b zeigt ein schematisches Ausführungsbeispiel des z-Wobbelns. Auch hier wird ein Laserstrahl 1 mittels einer Optik 2, 202 in einem transparenten Material 3 fokussiert. Der Fokus 4 wird dabei durch ein Scansystem entlang einer Scanlinie 5, die hier erzeugt wird durch eine periodisch wiederkehrende, schnelle Scanbewegung in z-Richtung, die mit einer langsamen Scanbewegung in einer x-y-Ebene überlagert ist, durch ein transparentes Augengewebe 3 bewegt. Eine solche Bewegung zur Bearbeitung von Augengewebe 3 mittels eines Laserscans erlaubt eine kleinere Optik 2, 202, da die schnellen Bewegungen jeweils in einem kleinen Volumen ausgeführt werden. Jedoch schatten bei ungünstigen Schnitt- bzw. Bearbeitungsmustern die schon erzeugten vertikalen Schnittlinien bzw. Bearbeitungslinien den Laserfokus 4 teilweise für dicht daneben anzubringende Schnittlinien ab.

Ziel ist es deshalb, eine Anordnung zur Bearbeitung einer Fläche in einem Bearbeitungsvolumen eines transparenten Materials zu beschreiben, bei dem mit einer kostengünstigen, also kleinen Optik gearbeitet werden kann und Abschattungswirkungen der bereits erfolgten Schnittlinien bzw. -muster im Laserfokus für noch zu erzeugende Schnittlinien bzw. -muster minimiert werden. Eine Möglichkeit bietet das laterale Wobbeln, wie es in den Druckschriften EP 2 412 341 B1 oder EP 2 596 773 B1 in einer x-y-Ebene offenbart wird.

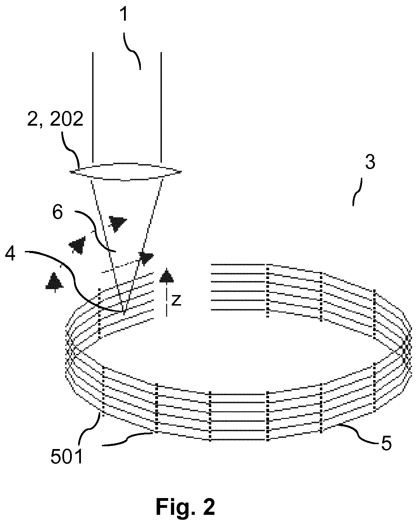

Ausgehend von solchen schnellen, beispielsweise oszillierenden Lateralbewegungen ist nun unter Hinzufügen einer langsamen z-Bewegung eine Erzeugung einer Ebene möglich, die parallel der z-Achse verläuft, wie sie in der Fig. 2 gezeigt wird. Ein Laserstrahl 1 oder eine andere Strahlung wird wiederum mittels einer Optik 2, 202 in einem transparenten Material 3 fokussiert. Der Fokus 4 wird dabei durch ein Scansystem entlang einer Scanlinie 5, die hier erzeugt wird durch eine periodisch wiederkehrende, schnelle laterale Scanbewegung, die mit einer langsamen Scanbewegung in einer z-Richtung überlagert ist, durch ein transparentes Augengewebe 3 bewegt. Dabei muss beispielsweise ein zylindrischer Schnitt mit signifikanter lokaler z-Ausdehnung, also einer Ausdehnung in z-Richtung, die größer ist als der Wirkbereich eines Laserfokus und somit mehrere übereinander angeordnete Scans mit dem Fokus 4 erfordert, und mit einer lateralen Ausdehnung, die größer ist als das (Teil-)Feld, das mit der Optik 2, 202 ohne zusätzliche laterale Bewegung gleichzeitig erreicht werden kann, aus mehreren "Flicken", also Teil-Bearbeitungsflächen, zusammengesetzt werden, um den Geschwindigkeitsvorteil des lateralen Wobbelns nutzen zu können. Ein solches Vorgehen des "Flickens" ist bei einem Einsatz des lateralen Wobbelns in der Augenheilkunde für eine Reihe von Behandlungsmustern, insbesondere von Schnittbildern wie beispielsweise dem zylindrischen Schnitt der Capsulotomie, nötig.

Ein laterales Wobbeln ist möglich mit einer Anordnung, die ein schnelles Scannen in einer senkrecht zu einer Strahlachse liegenden Ebene eines Bearbeitungsvolumens erlaubt.

Das Zusammensetzen aus mehreren Teil-Bearbeitungsflächen erzeugt allerdings viele Grenzbereiche 501, in denen zwei nebeneinanderliegende Teil-Bearbeitungsbereiche aneinander ausgerichtet werden müssen, damit einerseits keine "Bearbeitungslücke" entsteht und andererseits Bereiche nicht doppelt bearbeitet werden, für die dies nicht vorgesehen ist. Auch führt es in den Randbereichen der Teil-Bearbeitungsbereiche zu Situationen, in denen eine Abschattungswirkung durch bereits erzeugte Schnitt- bzw. Bearbeitungslinien unvermeidbar ist.

Aufgabe der vorliegenden Erfindung ist es deshalb, eine Anordnung, insbesondere für die Ophthalmologie, zur Bearbeitung einer Fläche in einem Bearbeitungsvolumen eines transparenten Materials mittels einer fokussierten Strahlung zu beschreiben, die ein Arbeiten mit einer kostengünstigen, also kleinen Optik erlauben, mit denen die Bearbeitung der Fläche jedoch in höchster Qualität erfolgt. Insbesondere sollen dabei Abschattungswirkungen der bereits erzeugten Bearbeitungslinien bzw. -muster, hier auch als Scanlinie bzw. Scanmuster bezeichnet, für noch zu erzeugende Bearbeitungslinien bzw. - muster vermieden oder zumindest minimiert werden, schwer aneinander ausrichtbare Grenzbereiche zwischen zwei Teil-Bearbeitungsflächen minimiert werden und Flächenformen mit größtmöglicher Flexibilität erzeugt werden. Die Gefahr von Schädigungen des bearbeiteten transparenten Materials soll ebenfalls minimiert werden.

Diese Aufgabe wird gelöst durch eine Anordnung zur Bearbeitung einer Fläche in einem Bearbeitungsvolumen eines transparenten Materials, insbesondere eines Auges, nach Anspruch 1 und 15, durch ein Steuerprogrammprodukt nach Anspruch 17 und durch eine Planungseinheit nach Anspruch 18.

Eine Anordnung ist ausgelegt zur Bearbeitung einer Fläche in einem Bearbeitungsvolumen eines transparenten Materials eines Auges, mittels einer fokussierten Strahlung. Die Anordnung ist demnach eine ophthalmologische Therapieanordnung, die in der Augenmedizin beispielsweise für augenchirurgische Zwecke oder aber zur Bestrahlung eingesetzt wird. Jedoch ist die Anordnung ihren Merkmalen und Funktionsprinzipien nach, wie aus den Erläuterungen erkennbar ist, nicht auf die Ophthalmologie beschränkt.

Die Bearbeitungsfläche kann eine zweidimensionale Fläche in einem dreidimensionalen Bearbeitungsvolumen sein. Die Fläche kann jedoch auch eine zusätzliche Krümmung aufweisen und somit eine Fläche bilden, die ein dreidimensionales Gebilde darstellt. Insbesondere kann die Fläche eine in sich geschlossene Fläche in einem dreidimensionalen Bearbeitungsvolumen sein.

Einen Spezialfall einer zu bearbeitenden Fläche bildet dabei die Ausbildung einer "Fläche" zur Erzeugung einer Bearbeitungslinie derart, dass diese "Fläche" in mindestens einer Raumrichtung signifikant ausgedehnt ist und in mindestens einer weiteren Raumrichtung eine Minimalausdehnung aufweist. Eine solche "Fläche" dient beispielsweise der Öffnung dünner Strukturen, in der Augenmedizin sei als ein Beispiel die Durchführung einer Capsulotomie genannt.

Die Bearbeitungsfläche kann eine Trennfläche bzw. Schnittfläche sein. Es kann sich jedoch auch um eine Fläche handeln, auf der das transparente Material abgetragen oder in seinen Materialeigenschaften verändert, beispielsweise verklebt, wird.

Ein transparentes Material ist dabei ein Material, das eine nur geringe, quasi vernachlässigbare, lineare Absorption im Wellenlängenbereich der eingesetzten Strahlung aufweist, so dass es einer zusätzlichen Maßnahme, beispielsweise der Fokussierung der Strahlung, bedarf, um eine Wirkung in diesem Material zu erzeugen.

Das zu bearbeitende transparente Material kann ein Augengewebe sein, wie beispielsweise die Hornhaut, die Linse, der Linsensack oder der Glaskörper. Es kann sich jedoch auch um ein künstliches Material handeln, insbesondere um ein künstliches Augenmaterial, beispielsweise eine Intraokularlinse (IOL), an der Korrekturen vorgenommen werden sollen. Das zu bearbeitende Material kann organischer wie auch anorganischer Natur sein.

Die Anordnung umfasst eine Einrichtung zur Erzeugung einer Strahlung. Dies kann eine entsprechende Lichtquelle oder eine andere Strahlungsquelle sein, die eine fokussierbare Strahlung aussendet. Insbesondere kann die Einrichtung zur Erzeugung einer Strahlung einen Laser umfassen, also eine Vorrichtung zur Lichtverstärkung durch stimulierte Emission von Strahlung. Eine solcher Laser erzeugt Licht hoher Intensität, meist aus einem engen Frequenzbereich, mit einer hohen räumlichen Kohärenz.

Vorteilhaft ist die Nutzung eines gepulsten Lasers, insbesondere eines Kurzpulslasers wie eines Femtosekunden-Lasers (fs-Laser) zur Erzeugung eines Femtosekunden-Laserstrahls oder eines Pikosekunden-Lasers. Ein gepulster Laser emittiert das Licht in zeitlich begrenzten Portionen, also nicht kontinuierlich. Er bietet eine hohe Energiedichte.

Hierbei kann ein solcher Femtosekunden-Laser oder Pikosekunden-Laser beispielsweise eine Wellenlänge aus dem Wellenlängenbereich von 200nm bis 2000nm Wellenlänge aufweisen: Wasser oder Augengewebe weisen eine geringe lineare Absorption in diesem Bereich auf und sind somit ein für die Strahlung eines Femtosekunden-Lasers oder Pikosekunden-Lasers transparentes Material.

Heute übliche, hier einsatzbare, Femtosekunden-Laser weisen eine Wellenlänge aus dem Bereich von 750nm bis 1100nm auf. Auch der Einsatz von Femtosekunden-Lasern, die eine Wellenlänge aus dem Bereich von 375nm bis 550nm bzw. aus dem Bereich von 250nm bis 367nm aufweisen, was einer Verdoppelung bzw. einer Verdreifachung der Frequenz der üblichen Femtosekunden-Laser entspricht, ist denkbar. Insbesondere wird hier beispielhaft eine Femtosekunden-Laser aus dem Wellenlängenbereich von 1020nm bis 1060nm eingesetzt.

Die Pulsdauer eines Femtosekunden- oder Pikosekunden-Lasers, der hier einsetzbar ist, ist vorteilhaft wählbar aus einem Pulsdauerbereich von 50fs bis 5ps. Insbesondere eine Pulsdauer aus einem Bereich von 100fs bis 1ps, und ganz besonders aus einem Bereich von 300fs bis 700fs ist hier bevorzugt.

Die Pulsenergie eines hier einsetzbaren Femtosekunden- oder Pikosekunden-Lasers liegt vorteilhaft in einem Pulsenergiebereich von 20nJ - 20µJ.

Die Laserpulsrepetitionsrate, also die Wiederholungsrate der Laserpulse, wird üblicherweise aus einem Bereich von 10kHz bis 10MHz gewählt, vorteilhaft ist eine Laserpulsrepetitionsrate aus einem Bereich von 100kHz bis 1MHz.

Die Anordnung umfasst weiterhin eine Optik zur Fokussierung, d.h. zur Bündelung, der Strahlung in einem Fokus im Bearbeitungsvolumen. Der Fokuskegel der so fokussierten Strahlung weist einen Fokussierwinkel auf. Dieser kennzeichnet den Öffnungswinkel des Fokus, also einen Divergenzwinkel, der prinzipiell auch durch die numerische Apertur ausgedrückt werden kann. Der Fokussierwinkel beschreibt den Winkel zwischen einer auf dem Kegelmantel des Fokuskegels verlaufenden Geraden und der optischen Achse. Der so definierte Fokussierwinkel entspricht damit dem halben Konuswinkel des Fokuskegels. Vorteilhafterweise wird der Fokuswinkel während der Laserbearbeitung, auch in verschiedenen Tiefen im zu bearbeitenden transparenten Material, annähernd konstant gehalten, da vom Fokusierwinkel auch die Fokusgröße und damit die benötigte Laserpulsenergie für den gewünschten Effekt abhängt.

Die Optik zur Fokussierung der kohärenten Strahlung in einem Fokus ermöglicht ein Bildfeld im Bearbeitungsvolumen mit einer durch die Optik bedingten Bildfeldgröße.

Zunächst ist es von Vorteil, mit einem großen Bildfeld arbeiten zu können, da dann das komplette Zielfeld und damit das komplette Bearbeitungsvolumen ohne Verschiebung der Optik erreichbar ist. Eine Vergrößerung der Optik führt jedoch zu steigenden Herstellungskosten dieser Optik und würde eine Anordnung zur Bearbeitung einer Fläche in einem Bearbeitungsvolumen eines transparenten Materials somit entscheidend verteuern.

Eine preisgünstige Anordnung zur Bearbeitung einer Fläche in einem Bearbeitungsvolumen weist deshalb eine Bildfeldgröße auf, die kleiner ist als die maximale Ausdehnung einer x-y-Ebene des Bearbeitungsvolumens. Erst durch die Bewegung der Optik und damit durch die Bewegung des Bildfelds wird die maximale Ausdehnung einer x-y-Ebene des Bearbeitungsvolumens erreicht.

Die fokussierte Strahlung weist eine Strahlachse auf. Sie ist die Symmetrieachse der fokussierten Strahlung und ihres Fokuskegels.

Die Anordnung umfasst weiterhin eine Einrichtung zur Veränderung der Lage des Fokus im Bearbeitungsvolumen, das durch drei Raumrichtungen x, y und z beschreibbar ist. Dabei verlaufen die x-Richtung und die y-Richtung als Lateralrichtungen nicht parallel zueinander und jeweils senkrecht zu einer Grundstellungs-Strahlachse, und die z-Richtung verläuft parallel zur Grundstellungs-Strahlachse, wobei die Grundstellungs-Strahlachse die Strahlachse der fokussierten Strahlung ohne eine Auslenkung des Fokus durch die Einrichtung zur Veränderung der Lage des Fokus im Bearbeitungsvolumen in den beiden Lateralrichtungen x und y kennzeichnet.

Die Veränderung der Lage des Fokus erfolgt bevorzugt kontinuierlich, jedoch nicht notwendigerweise mit einer konstanten Geschwindigkeit.

Auch ein durch die Kreiskoordinaten R und Φ und die parallel zur Grundstellungs-Strahlachse verlaufenden Richtung z beschriebenes Bearbeitungsvolumen oder aber ein durch Kugelkoordinaten beschriebenes Bearbeitungsvolumen ist dabei ein durch drei Raumrichtungen x, y und z beschreibbares Bearbeitungsvolumen: Alle dreidimensionalen Koordinatensysteme sind stets ineinander umrechenbar.

Die Bearbeitung einer Fläche im Bearbeitungsvolumen wird durch die Veränderung der Lage des Fokuspunkts der fokussierten Strahlung innerhalb des Bearbeitungsvolumens erreicht. Am Ort des Fokuspunktes der fokussierten Strahlung entsteht ein Wirkbereich dieser fokussierten Strahlung im transparenten Material, der als Fokuswirkbereich bezeichnet wird: Am und in einer nächsten Umgebung um den Fokuspunkt herum verändert der fokussierte Strahlung das für eine entsprechende unfokussierte Strahlung transparente Material.

Beispielsweise entstehen bei Einsatz einer gepulsten Laserstrahlung und einer konstanten Geschwindigkeit der Veränderung der Fokuslage, der Scangeschwindigkeit, Fokuswirkbereiche in einem gleichmäßigen Abstand, der von der Pulsrate bestimmt ist. Bei einer variablen Scangeschwindigkeit und einer konstanten Pulsrate der gepulsten Laserstrahlung variiert der Abstand der Fokuswirkbereiche.

In der Regel enthält eine Bearbeitungsfläche eine Vielzahl von über die gesamte Fläche verteilten Fokuswirkbereichen, die sich maximal in einem solchen Abstand voneinander befinden, dass die beabsichtigte Wirkung durch den Einfluss der fokussierten Strahlung nicht unterbrochen wird. Dies führt zum entsprechenden Bearbeitungsresultat entlang der zu bearbeitenden Fläche, wie zu einem "Schnitt" durch Trennung des transparenten Materials, beispielsweise durch Photodisruption, zu einer Abtragung des Materials durch Ablation, oder zu einer Veränderung des Materials, beispielsweise einer Verklebung durch Koagulation des Materials in den Fokuswirkbereichen einer gepulsten Laserstrahlung.

Die Anordnung bietet die Möglichkeit, ein transparentes Material innerhalb des Bearbeitungsvolumens zu bearbeiten. Das Bearbeitungsvolumen stellt also den Raumbereich dar, innerhalb dessen die Anordnung aufgrund Ihrer Möglichkeiten zur räumlichen Veränderung des Fokuspunktes der fokussierten Strahlung, der somit einen beweglichen Arbeitspunkt darstellt, die Bearbeitung des Materials vornehmen kann. Das zu bearbeitende transparente Material bzw. der zu bearbeitende Teil des transparenten Materials befindet sich dabei idealerweise komplett im Bearbeitungsvolumen.

Die Anordnung umfasst zudem eine Steuereinrichtung, die eingerichtet ist zur Steuerung der Anordnung, insbesondere zur Steuerung der Einrichtung zur Erzeugung einer Strahlung, der Einrichtung zur Veränderung der Lage des Fokus der fokussierten Strahlung und/oder der Optik. Sie ist hierzu über entsprechende Kommunikationswege mit der Einrichtung zur Erzeugung einer Strahlung, der Einrichtung zur Veränderung der Lage des Fokus der fokussierten Strahlung und/oder der Optik verbunden.

Die Steuereinrichtung kann einteilig oder mehrteilig ausgeführt sein. In einer einteiligen Ausführung enthält sie einen Controller, von dem aus alle Teile der Anordnung kontrolliert und/oder gesteuert werden. Ist sie mehrteilig ausgeführt, so kann sie mehrere Kontroll- und Steuereinheiten enthalten, die miteinander in Verbindung stehen.

Die Steuereinrichtung kann mit einem Steuerprogrammprodukt kodiert sein oder mit einem Steuerprogrammprodukt kodiert werden, das sich entweder auf einem Datenträger befindet, wobei der Datenträger zum Zwecke der Kodierung mit der Steuereinrichtung in Verbindung gebracht wird, oder aber das Steuerprogrammprodukt wird im Internet oder an einem sonstigen externen Speicherplatz zum Download zur Verfügung gestellt, wobei die Steuereinrichtung eine Verbindung zum Internet oder zum sonstigen externen Speicherplatz direkt oder über Zwischenstufen herstellen kann.

Dabei kann eine Planungseinheit als abgrenzbare Teileinheit in der Steuereinrichtung enthalten sein, die eingerichtet ist, alle Schritte der Planung bzw. Bereitstellung einer Therapieführung, insbesondere von Parametern der Strahlungserzeugung, der Fokussierung sowie von Scanbewegungen zur Bearbeitung einer Fläche, im zeitlichen Verlauf auszuführen. Die Planungseinheit ist damit eingerichtet, ein Scanmuster, als zeitliche Änderung der Lage eines Fokus der Strahlung und damit von Fokuswirkbereichen im zu bearbeitenden Material, zu planen und/oder bereitzustellen.

Bei einer mehrteiligen Steuereinrichtung kann eine solche Planungseinheit auch eine von anderen Einheiten der Steuereinrichtung unabhängige Einheit darstellen, die über Kommunikationswege mit den anderen Einheiten der Steuereinrichtung verbunden ist.

In einer Ausgestaltung kann die Planungseinheit eine Auswahltabelle von Scanmustern enthalten. In einer anderen Ausgestaltung enthält sie einen Algorithmus zur Erstellung eines Scanmusters.

Wird eine Steuereinrichtung kodiert, die eine solche Planungseinheit enthält, so werden die Scanbewegungen, insbesondere das Scanmuster, von dieser Planungseinheit bereitgestellt.

Erfindungsgemäß ist die Einrichtung zur Veränderung der Lage des Fokus der Anordnung ausgebildet, in jeder beliebigen, durch die drei Raumrichtungen bestimmten Richtung eine langsame Scanbewegung im Bearbeitungsvolumen des transparenten Materials und - ebenfalls in jeder beliebigen, durch die drei Raumrichtungen bestimmte Richtung - eine von der langsamen Scanbewegung unabhängige schnelle Scanbewegung in einem Teilbereich des Bearbeitungsvolumens zu vollziehen. Dabei ist der Teilbereich der schnellen Scanbewegung durch die langsame Scanbewegung im gesamten Bearbeitungsvolumen bewegbar. Es arbeitet also ein komplettes schnelles dreidimensionales Scansystem mit einem von diesem unabhängigen, langsamen dreidimensionalen Scansystem so miteinander, dass das schnelle dreidimensionale Scansystem jeweils einen Teilbereich bzw. ein Teilvolumen des gesamten Bearbeitungsvolumens erreicht. Dieser Teilbereich kann jedoch unter Zuhilfenahme des langsamen dreidimensionalen Scansystems durch das gesamte Bearbeitungsvolumen bewegt werden.

Die mögliche Größe des Teilbereichs der schnellen Scanbewegung wird von der Bildfeldgröße der eingesetzten Optik, aber auch von der Art der Erzeugung der schnellen Scanbewegung bestimmt.

Die Begriffe "langsam" und "schnell" definieren sich hier zueinander und werden eingesetzt zur Kennzeichnung der beiden grundsätzlichen Scanbewegungen und Scansysteme. Die schnelle Scanbewegung erfolgt mit einer Maximal-Geschwindigkeit, die ein Vielfaches der Maximal-Geschwindigkeit der langsamen Scanbewegung beträgt. Vorzugsweise kann die schnelle Scanbewegung ein Zehnfaches bis ein Tausendfaches der langsamen Scanbewegung betragen. Vorteilhaft ist eine schnelle Scanbewegung, die etwa ein Hundertfaches der langsamen Scanbewegung beträgt.

Typische Größen des Bearbeitungsvolumens, in dem eine langsame Scanbewegung erfolgt, können für eine Bearbeitungseinrichtung, die beispielsweise als ophthalmologische Therapievorrichtung eingesetzt wird, in jeder Raumrichtung zwischen 2mm und 25mm, vorzugsweise zwischen 5mm und 12mm, liegen. Dieses Volumen kann typischerweise mit Geschwindigkeiten aus einem Bereich von 1 bis 100 mm/s, vorzugsweise von ca. 10mm/s gescannt werden.

Eine typische Größe eines durch die schnelle Scanbewegung gleichzeitig erreichbaren Teilbereichs liegt in jeder Raumrichtung zwischen 0,5mm und 2mm. Beispielsweise kann durch die schnelle Scanbewegung ein dreidimensionaler Teilbereich von ca. 1mm x 1mm x 1mm erreicht werden. Dabei sind Scanbewegungen mit mehreren hundert Hertz möglich.

Die beliebige, veränderbare, durch die drei Raumrichtungen bestimmte Richtung ergibt sich sowohl für die schnelle als auch für die langsame Scanbewegung durch Vektoraddition von den jeweiligen Scan-Anteilen in den drei Raumrichtungen x, y und z pro Zeiteinheit für die schnelle Scanbewegung wie auch für die langsame Scanbewegung. Durch Vektoraddition der Richtung der schnellen und der Richtung der langsamen Scanbewegung für dieselbe Zeiteinheit ergibt sich wiederum eine Gesamtrichtung der beiden Scanbewegungen für diese Zeiteinheit.

Die erfindungsgemäße Anordnung erlaubt es also einerseits, einen Teilbereich des Bearbeitungsvolumens mit hoher Geschwindigkeit und das gesamte Bearbeitungsvolumen mit einer signifikant geringeren Geschwindigkeit zu scannen: Ein sich mit einer langsamen Scanbewegung in einer beliebigen und im Laufe des Scanvorgangs veränderbaren Richtung entsprechen eines gewünschten Musters durch das Bearbeitungsvolumen bewegender Fokus der fokussierten Strahlung unterliegt damit im Laufe des Scanvorgangs einer zusätzlichen schnellen Scanbewegung in einer zweiten beliebigen veränderbaren Richtung, die der langsamen Scanbewegung überlagert ist. Diese zusätzliche schnelle Scanbewegung wird allerdings immer nur in einen kleinen Teilbereich des Bearbeitungsvolumens ermöglicht.

Die hohe Geschwindigkeit, mit der die schnelle Scanbewegung ausgeführt wird, erlaubt zudem, bei einem Einsatz eines gepulsten Lasers aus dem Pikosekundenbereich bzw. Femtosekundenbereich, der eine hohe Laserpulsrepetitionsrate aufweist, die Fokuswirkbereiche entsprechend im Bearbeitungsvolumen zu verteilen und nicht etwa zu nah zueinander zu positionieren.

Diese "Arbeitsteilung" zwischen langsamer und schneller Scanbewegung erlaubt es, für die schnelle Scanbewegung Scansysteme zu nutzen, die fähig sind, die Lage des Fokus extrem schnell zu verändern, da sie keine großen, schweren Optik bewegen müssen, während für die langsame Scanbewegung mit anderen Scansystemen die Optik bewegt werden kann.

Eine Anordnung, insbesondere eine ophthalmologische Therapieanordnung, umfasst eine Steuereinrichtung, in die ein Scanmuster kodiert ist, das eine Abfolge von Fokuswirkbereichen der fokussierten Strahlung entlang einer Scanlinie aufweist. Die Abfolge von Fokuswirkbereichen ist dabei in vorteilhafter Ausgestaltung der erfindungsgemäßen Anordnung so kodiert, dass bereits realisierte Fokuswirkbereiche stets außerhalb eines Fokuskegels, der durch den Fokus der fokussierten Strahlung und den Fokussierwinkel beschrieben ist, für noch zu realisierende Fokuswirkbereiche angeordnet sind.

Dabei ist die Scanlinie die Linie bzw. Spur, die entsteht oder entstehen würde, wenn der Fokus der fokussierten Strahlung, und damit auch der Fokuskegel, mit Hilfe der Einrichtung zur Veränderung der Lage des Fokus im Bearbeitungsvolumen bewegt wird, und es sich um eine kontinuierliche Strahlung handelt bzw. handelt würde. Die Scanlinie kann eine Gerade sein, oder aber auch ganz oder teilweise in einer Ebene oder in einem dreidimensionalen Raum gekrümmt sein.

Die Scanlinie beschreibt also den während des Scanvorgangs in Abhängigkeit von der Zeit erreichten Ort des Fokus im Bearbeitungsvolumen, unabhängig davon, ob die fokussierte Strahlung gerade zur Wirkung kommt oder nicht. Entlang der Scanlinie werden dann die tatsächlichen Fokuswirkbereiche der fokussierten Strahlung gesetzt.

Wird also beim Scannen der Fokuskegel auf einer Scanlinie derart durch das Bearbeitungsvolumen geführt, dass der Fokuskegel niemals den schon zurückgelegten Teil der Scanlinie schneidet, dann ist diese Bedingung, dass bereits realisierte Fokuswirkbereiche stets außerhalb eines Fokuskegels für noch zu realisierende Fokuswirkbereiche angeordnet sind, auf jeden Fall erfüllt. Die Bedingung kann aber auch noch erfüllt sein, wenn es beim Scannen zwar zu Positionen kommt, in denen ein ggf. gedachter Fokuskegel den bereits zurückgelegten Teil der Scanlinie schneidet: Dies ist dann der Fall, wenn in dem geschnittenen Bereich bzw. in den geschnittenen Bereichen der Scanlinie keine Fokuswirkbereiche realisiert wurden, oder aber ein geschnittener Bereich zwar realisierte Fokuswirkbereiche enthält, die fokussierte Strahlung aber solange ausgeblendet bleibt, wie sich ihr gedachter Fokuskegel im Bereich bereits realisierter Fokuswirkbereiche befindet.

In einfacher Art und Weise ist diese Bedingung erfüllt, wenn ein Scanmuster so aufgebaut wird, dass zunächst die Fokuswirkbereiche appliziert werden, die sich im Strahlengang der Strahlung am weitesten von der Einrichtung zur Erzeugung der Strahlung entfernt befinden: Die weiteren Fokuswirkbereiche werden dann sukzessive in Richtung größerer z-Werte, also zur Strahlungsquelle hin, realisiert. Soll also ein Scanmuster in einem Bereich entstehen, der kleiner als der Teilbereich ist, in dem die schnelle Scanbewegung vollzogen werden kann, so kann das Scanmuster auf diese Art und Weise kodiert und realisiert sein. Auch ist dies eine Lösung, wenn ein nächstes Scanmuster, bzw. ein nächster Teil eines komplexen Scanmusters in einem Abstand zu diesem ersten Scanmuster des ersten Teilbereichs entstehen soll, bei dem der Fokuskegel der fokussierten Strahlung für die zu realisierenden Fokuswirkbereiche des Scanmusters im zweiten Teilbereich nicht bereits realisierte Fokuswirkbereiche des Scanmusters des ersten Teilbereichs umfasst, was der Fall wäre, wenn das zweite Scanmuster bzw. der zweite Teil eines komplexen Scanmusters nicht direkt in Anschluss an den ersten gesetzt werden soll.

Sollen jedoch beide Teilbereiche, insbesondere über einen weiten z-Bereich, direkt nebeneinander platziert werden, etwa weil die Bearbeitungsfläche nahtlos fortgeführt werden soll, dann werden zumindest in einem Grenzbereich einige bereits realisierte Fokuswirkbereiche im Fokuskegel noch zu realisierender Fokuswirkbereiche angeordnet sein. Sollen auch diese vermieden werden, so müssen diese Grenzbereiche zunächst in Abhängigkeit von der Größe des Fokussierwinkels des Fokuskegels der fokussierten Strahlung ausgelassen werden und am Ende sukzessive aufgefüllt werden.

Vorzugsweise enthält die Anordnung eine Einrichtung zur Veränderung der Lage des Fokus, die einen schnellen z-Scanner zum schnellen Scannen in einer Richtung parallel zur Grundstellungs-Strahlachse und mindestens einen schnellen Lateralscanner zum schnellen Scannen in einer Ebene senkrecht der Grundstellungs-Strahlachse zur Realisierung der schnellen Scanbewegung im Teilbereich des Bearbeitungsvolumens zusätzlich zu einem langsamen z-Scanner zum langsamen Scannen in einer Richtung parallel zur Grundstellungs-Strahlachse und mindestens einem langsamen Lateralscanner zum langsamen Scannen in einer Ebene senkrecht der Grundstellungs-Strahlachse zur Realisierung der langsamen Scanbewegung im Bearbeitungsvolumen umfasst.

Durch ein in Abhängigkeit von einer gewünschten Richtung der schnellen Scanbewegung gesteuertes Zusammenwirken eines oder mehrerer schneller Lateralscanner und des schnellen z-Scanners ist somit eine schnelle Scanbewegung in einer beliebigen, änderbaren Richtung in einem Teilbereich des Bearbeitungsvolumens möglich.

Für die langsame Scanbewegung im Bearbeitungsvolumen stehen hingegen ein vom schnellen z-Scanner unabhängiger langsamer z-Scanner und ein oder mehrere langsame Lateralscanner, die von den jeweiligen schnellen Lateralscannern ebenfalls unabhängig sind, zur Verfügung, deren Zusammenwirken ebenfalls entsprechend der gewünschten Richtung gesteuert wird.

In einer Ausführungsform der Anordnung ist der schneller Lateralscanner durch einen schnellen x-Scanner ausgebildet, der neben einem weiteren schnellen Lateralscanner vorliegt. Dieser weitere schnelle Lateralscanner ist durch einen schnellen y-Scanner ausgebildet. Der schnelle x-Scanner und der schnelle y-Scanner können dabei auch in einer gemeinsamen x-y-Scaneinheit vorliegen. Eine solche x-y-Scaneinheit, die faktisch einen schnellen x-Scanner und einen schnellen y-Scanner enthält, kann beispielsweise durch einen kardanischen Scanner realisiert sein.

In einer alternativen Ausführungsform ist ein schneller Lateralscanner durch einen schnellen R-Scanner ausgebildet, dessen Scanbewegung in einer x-y-Ebene senkrecht zur Grundstellungs-Strahlachse durch Drehung um eine zur Grundstellungs-Strahlachse parallele Drehachse um einen Winkel Φ ausrichtbar ist. Die Drehung selbst kann dabei langsam erfolgen.

Gleiche alternative Ausführungsformen sind prinzipiell auch für den oder die langsamen Lateralscanner möglich.

Das langsame wie auch das schnelle Lateralscannen kann sich also zusammensetzen aus den Bewegungen eines x- und eines y-Scanners, wobei die Bewegungen des x-Scanner und des y-Scanner so aufeinander abgestimmt sind, dass dabei eine gewünschte Richtung der Scanbewegung in der x-y-Ebene entsteht. Alternativ kann das Lateralscannen in einer gewünschten Richtung der Scanbewegung in der x-y-Ebene durch einen R-Scanner erfolgen, wobei die Richtung der Scanbewegung dadurch gesteuert wird, dass der R-Scanner durch Drehung um einen Winkel Φ um eine zur Grundstellungs-Strahlachse parallele Drehachse ausgerichtet wird.

In einer besonderen Ausführungsform enthält die Anordnung einen schnellen z-Scanner, der ein in z-Richtung oszillierendes Linsenelement umfasst. Dieses oszillierende Linsenelement ist vorzugsweise eine negative Linse, also eine Konkavlinse, die zusammen mit einer fester Positivlinse, also einer Konvexlinse, als Strahlaufweitungs-Teleskop agiert. Als oszillierendes Linsenelement kann prinzipiell auch eine positive Linse, also eine Konvexlinse, gewählt werden. Dann ist allerdings auf die Lage eines Zwischenfokus zu achten.

In dieser Ausführungsform enthält die Anordnung weiterhin einen schnellen zweidimensionalen Lateralscanner, der ein um zwei Achsen bewegliches x-y-Spiegelelement oder zwei einzelne jeweils um eine Achse bewegliche Spiegelelemente, deren Achsen bevorzugt senkrecht zueinander stehen, und damit eine beliebige Bewegungsrichtung in einer lateralen Ebene ermöglichen, umfasst. Durch diesen schnellen Lateralscanner ist eine schnelle Bewegung in der lateralen Ebene, also der x-y-Ebene, insbesondere eine schnelle oszillierende Bewegung in der x-y-Ebene, möglich.

In dieser Ausführungsform enthält die Anordnung auch einen langsamen z-Scanner, der ein in z-Richtung wahlfrei bewegliches Linsenelement umfasst, wiederum vorzugsweise eine negative Linse, die mit einer fester Positivlinse als Strahlaufweitungs-Teleskop agiert, und einen langsamen Lateralscanner, der eine wahlfrei in der lateralen Ebene verschiebbare Fokussieroptik umfasst. Die Optik zur Fokussierung ist also lateral verschiebbar, wobei die gerichtete Strahlung durch mit der verschiebbaren Fokussieroptik in fester Beziehung stehende, mitlaufende Spiegel auf diese Fokussieroptik gelenkt wird.

In einer Ausführungsform der Anordnung erfolgen synchrone Richtungswechsel der schnellen Scanbewegung in mindestens zwei Raumrichtungen. Dies wird durch synchrone Richtungswechsel mindestens zweier schneller Scanner ausgeführt. Dabei erfolgen die synchronen Richtungswechsel mindestens zweier schneller Scanner in einer definierten zeitlichen Beziehung zueinander. Insbesondere können sie gleichzeitig erfolgen. Alternativ ist aber auch ein definierter zeitlicher Versatz der synchronen Richtungswechsel mindestens zweier schneller Scanner möglich.

Die schnellen Scanner fahren also vorbestimmte Orts-Zeit-Kurven ab, die aufeinander synchronisiert sind. Es ist nicht unbedingt erforderlich, dass diese auf die Bewegung der langsamen Scanner synchronisiert sind.

Die synchronen Richtungswechsel der schnellen Scanbewegung in mindestens zwei Raumrichtungen können dabei periodisch erfolgen und durch synchrone periodische Richtungswechsel mindestens zweier schneller Scanner ausgeführt werden. Die schnellen Scanner in den entsprechenden Raumrichtungen wechseln also ihre Richtung zeitlich aufeinander abgestimmt in einer definierten Periode.

Insbesondere kann dies auf einfache Art und Weise durch synchrone oszillatorische Bewegungen mindestens zweier schneller Scanner ausgeführt werden.

Eine synchrone oszillatorische Bewegung zweier schneller Lateralscanner, also eines schnellen x-Scanners und eines schnellen y-Scanners, ermöglicht die Bearbeitung einer Fläche senkrecht zur Grundstellungs-Strahlachse. Überschreitet diese den erreichbaren Teilbereich des Bearbeitungsvolumens, der gleichzeitig durch die schnellen Scanner erreicht werden kann, so ist eine Überlagerung einer langsamen Scanbewegung ein x- und/oder y-Richtung nötig, um eine solche Fläche zu bearbeiten. Werden oszillatorische Bewegungen zweier schneller Lateralscanner, die aufeinander synchronisiert sind, mit einer langsamen Scanbewegung überlagert, die auch eine z-Komponente enthält, dann sind Bearbeitungsflächen wie in der Fig. 2 gezeigt möglich.

Einer dieser schnellen Scanner kann vorteilhaft ein resonanter Scanner sein, die anderen schnellen Scanner müssen sich dann auf dessen Resonanzfrequenz synchronisieren lassen. Das heißt also, dass dann einer der mindestens zwei schnellen Scanner ein resonanter Scanner mit einer freien Oszillation ist, und alle weiteren der mindestens zwei schnellen Scanner auf den resonanten Scanner synchronisiert sind. Diese Synchronisation erfolgt vorteilhaft phasenstarr.

Durch den Einsatz eines resonanten Scanners ist es möglich, die Scanfrequenz und damit die Scangeschwindigkeit im Vergleich zu einem reinem Einsatz nichtresonanter Scanner signifikant zu erhöhen, ein Faktor 5 bis 10 ist hierbei möglich.

Besonders bevorzugt ist die Ausführungsform, in der die schnelle Scanbewegung durch synchrone oszillatorische Bewegungen des schnellen z-Scanners und mindestens eines schnellen Lateralscanners, also eines schnellen x- und/oder y-Scanners oder aber des R-Scanners ausgeführt ist.

Beispielsweise werden in der erfindungsgemäßen Anordnung insgesamt sechs Scanner eingesetzt. Für jede Raumrichtung x, y und z gibt es dann jeweils einen schnellen Scanner, der oszillierende Bewegungen ausführt. Dies erlaubt, einen dreidimensionalen Teilbereich des Bearbeitungsvolumens von ca. 1mm x 1mm x 1mm mit mehreren hundert Hertz zu scannen. Zusätzlich gibt es für jede Raumrichtung x, y und z einen langsamen Scanner, was erlaubt, das komplette nötige Bearbeitungsvolumen zu erreichen.

Zur vorteilhaften Bearbeitung einer Fläche in einem Bearbeitungsvolumen wird in einer erfindungsgemäßen Anordnung, die eine schnelle Scanbewegung unabhängig von einer langsamen Scanbewegung in jede beliebige Richtung im Bearbeitungsvolumen vollziehen kann, und die sich zur Realisierung der schnellen Scanbewegung der synchronen oszillatorischen Bewegungen mindestens zweier schneller Scanner, und insbesondere der synchronen oszillatorischen Bewegungen eines schnellen z-Scanners und mindestens eines, in der Regel zweier schneller Lateralscanner in Form eines x-Scanners und eines y-Scanners, bedient, die Bewegung zur Realisierung eines Scanmusters entlang der Scanlinie aus einer langsamen Scanbewegung und einer schnellen Scanbewegung durch die synchron miteinander oszillierenden schnellen Scanner zusammengesetzt.

Dabei kann die langsame Scanbewegung mit konstanter Geschwindigkeit oder aber in Abhängigkeit vom Ort und/oder Zeit variierender Geschwindigkeit vollzogen werden. Sie weist eine Lateralkomponente in x-Richtung und/oder in y-Richtung auf.

Es ist das Ziel, ein Scanmuster derart zu erstellen, dass ein bereits realisierter Fokuswirkbereich stets außerhalb des Fokuskegels eines noch zu realisierenden Fokuswirkbereich angeordnet ist. Deshalb ist es wichtig, dass eine oszillierende, also "schwingende" Scanbewegung so vollführt wird, dass minimale z-Werte erreicht werden, wenn sie in langsamer "Vorschubrichtung" vorausschwingt. So werden noch zu realisierender Fokuswirkbereiche stets über bereits realisierten Fokuswirkbereichen platziert.

Die oszillatorische Bewegung des schnellen z-Scanners ist deshalb zur oszillatorischen Bewegung eines schnellen Lateralscanners oder zweier schneller Lateralscanner in Form eines schnellen x- und/oder schnellen y-Scanners so synchronisiert, dass bei einer positiven Lateralkomponente der langsamen Scanbewegung in x- und/oder y-Richtung die oszillatorischen Bewegungen des schnellen Lateralscanners, insbesondere des schnellen x-Scanners und/oder des schnellen y-Scanners, gegenphasig zur oszillatorischen Bewegung des schnellen z-Scanners sind, und dass bei einer negativen Lateralkomponente der langsamen Scanbewegung in x- und/oder y-Richtung die oszillatorischen Bewegungen des schnellen Lateralscanners, insbesondere des schnellen x-Scanners und/oder des schnellen y-Scanners, gleichphasig zur oszillatorischen Bewegung des schnellen z-Scanners sind.

Die oszillatorische Bewegung des schnellen z-Scanners zwischen einem minimalen z-Wert und einem maximalen z-Wert ist also synchronisiert zur oszillatorischen Bewegung eines schnellen Lateralscanners oder zweier schneller Lateralscanner in Form eines schnellen x- und/oder schnellen y-Scanners, die zwischen einem minimalen x- Wert und einem maximalen x- Wert und/oder einem minimalen y-Wert und einem maximalen y-Wert oszillieren. Die Amplitude der oszillatorischen Bewegung kann sich dabei zeitlich bzw. örtlich ändern. Die maximalen und minimalen x, y bzw. z-Werte der oszillatorischen Bewegung in einem Teilbereich des Bearbeitungsvolumens sind also nicht als Konstante anzusehen.

Dabei sind die mindestens zwei oszillatorischen Bewegungen derart zueinander synchronisiert, dass
- der maximale x-Wert des schnellen Lateralscanners in x-Richtung, also des schnellen x-Scanners, und/oder der maximale y-Wert des schnellen Lateralscanners in y-Richtung, also des schnellen y-Scanners, bei einem minimalen z-Wert des schnellen z-Scanners sowie der minimale x- Wert des schnellen Lateralscanners in x-Richtung und/oder der minimale y-Wert des schnellen Lateralscanners in y-Richtung bei einem maximalen z-Wert des schnellen z-Scanners für eine Lateralkomponente der langsamen Scanbewegung in positive x- und/oder y-Richtung, und
- der minimale x-Wert des schnellen Lateralscanners in x-Richtung und/oder der minimale y-Wert des schnellen Lateralscanners in y-Richtung bei einem minimalen z-Wert des schnellen z-Scanners sowie der maximale x-Wert des schnellen Lateralscanners in x-Richtung und der maximale y-Wert des schnellen Lateralscanners in y-Richtung bei einem maximalen z-Wert des schnellen z-Scanners für eine Lateralkomponente der langsamen Scanbewegung in negative x- und/oder y-Richtung auftritt.

Sobald es also eine Lateralkomponente der langsamen Scanbewegung in dieselbe Richtung gibt, in der eine oszillatorische Bewegung eines schnellen Lateralscanners für die Realisierung der schnellen Scanbewegung vorliegt, und zudem eine oszillatorische Bewegung eines schnellen z-Scanners vorliegt, so muss bei der Synchronisation der schnellen Scanner untereinander auf die genannten Bedingungen geachtet werden: Dies ermöglicht es, ein Scanmuster so zu realisieren, dass Fokuswirkbereiche auf geneigten Scanlinien entstehen und bereits realisierte Fokuswirkbereiche stets außerhalb eines Fokuskegels, der durch den Fokus der fokussierten Strahlung und den Fokussierwinkel gebildet ist, für noch zu realisierende Fokuswirkbereiche angeordnet sind.

Die langsame Scanbewegung kann dabei auch eine Komponente in z-Richtung aufweisen. Bezüglich einer langsamen Scanbewegung in z-Richtung ist jedoch die Synchronisation zweier schneller Bewegungen mit periodischem Richtungswechsel, insbesondere die Phasenlage der Synchronisation der oszillatorischen Bewegung eines schnellen z-Scanners mit der oszillatorischen Bewegung mindestens eines schnellen Lateralscanners nicht von Bedeutung. Die langsame Scanbewegung in z-Richtung muss hingegen immer in positiver Richtung, also parallel zur Grundstellungs-Strahlachse und entgegen der Strahlrichtung der Strahlung, erfolgen.

Weiterhin vorteilhaft ist eine Anordnung, die eine Steuereinrichtung umfasst, in die ein Scanmuster kodiert ist, das zueinander benachbarte Strokes umfasst. Ein Stroke umfasst einen im wesentlichen gleichgerichteten Teil einer Scanlinie, und ist durch eine Aneinanderreihung von Fokuswirkbereichen der fokussierten Strahlung auf diesem Teil der Scanlinie realisiert.

Ein Stroke muss dabei nicht notwendigerweise eine Gerade beschreiben: Es sind Krümmungen bzw. Abweichungen von einer Geraden von mehreren Grad bis mehreren zehn Grad möglich. Der Teil der Scanlinie, den ein Stroke umfasst, ist vielmehr in eine Richtung im Bearbeitungsvolumen der Anordnung ausgerichtet, so dass er keine Umkehrpunkte der schnellen Scanbewegung in x-, y- und/oder z-Richtung umfasst. Passiert die Scanlinie einen Umkehrpunkt der schnellen Scanbewegung, so entsteht hernach ein nächster Stroke.

Zueinander benachbarte Strokes weisen einen im wesentlichen gleichen Abstand auf. Dass der Abstand nicht exakt gleich bleiben muss bedeutet, dass der Abstand zueinander benachbarte Strokes durchaus um mehrere Prozent oder sogar mehrere zehn Prozent schwanken kann, wobei ein maximaler Abstand nicht überschritten werden sollte, so dass die beabsichtigte Wirkung durch den Einfluss der fokussierten Strahlung nicht unterbrochen wird. Schon allein durch eine sich verändernde Krümmung oder Ausrichtung eines Strokes im Vergleich zum benachbarten Stroke ist ein exakt gleicher Abstand zweier benachbarter Strokes über ihre gesamte Länge meist nicht möglich.

Die Strokes weisen jeweils Neigungswinkel auf, wobei der Neigungswinkel eines Strokes der Winkel zwischen dem Stroke und der Strahlachse ist. Die Neigungswinkel zweier benachbarte Strokes müssen nicht gleich sein, auch entlang eines Strokes kann sich der Neigungswinkel ändern. Für einen gekrümmten Stroke ist der entscheidende Neigungswinkel der kleinste Winkel zwischen einer Tangente an die Krümmungslinie des Stroke und der Strahlachse.

Das Scanmuster muss jedoch in dieser Ausführungsform der erfindungsgemäßen Anordnung derart in die Steuereinrichtung kodiert sein, dass der jeweilige Neigungswinkel des Strokes zur Strahlachse größer oder gleich dem Fokussierwinkel der fokussierten Strahlung ist.

Die Bedingung, dass der Neigungswinkel des Strokes zur optischen Achse größer sein muss als der Fokussierwinkel der fokussierten Strahlung, gilt dabei für jeden einzelnen Stroke und an jeder Stelle des Strokes - letztere Bedingung ist wichtig für gekrümmte Strokes. Die Bedingung ist durch die Betrachtung des kleinsten Winkels, wie oben dargestellt, erfüllt.

Unabhängig von ihrem Neigungswinkel wird für in Richtung der Strahlachse und entgegen der Strahlrichtung der Strahlung übereinanderliegende Strokes oder sich überkreuzende Strokes immer ein folgendes Stroke über dem benachbarten vorangegangenen Stroke realisiert, damit die Bedingung, dass bereits realisierte Fokuswirkbereiche stets außerhalb eines Fokuskegels für noch zu realisierende Fokuswirkbereiche angeordnet sind, erfüllt ist.

Werden die Strokes durch die Überlagerung einer langsamen und einer schnellen Scanbewegung erzeugt, wobei die schnelle Scanbewegung durch synchrone oszillatorische Bewegungen eines schnellen z-Scanners und mindestens eines schnellen Lateralscanners realisiert ist, so sind die genannten Bedingungen bzgl. minimaler und maximaler Werte für die Synchronisation zu beachten; der notwendige Neigungswinkel kann durch eine entsprechendes Verhältnis von langsamer und schneller Scanbewegung erzeugt werden.

Dabei ist in einer Variante einer erfindungsgemäßen Anordnung die Steuereinrichtung derart kodiert, dass die Ausbildung eines Strokes durch eine Aneinanderreihung von Fokuswirkbereichen der fokussierten Strahlung immer nur in einer Aufwärtsbewegung entlang der Scanlinie oder immer nur in einer Abwärtsbewegung entlang der Scanlinie realisiert ist: Eine Aufwärtsbewegung ist dabei eine Bewegung mit einer z-Komponente entgegen der Strahlrichtung, während eine Abwärtsbewegung eine Bewegung mit einer z-Komponente in Richtung der Strahlrichtung ist.

Werden die Strokes durch synchrone oszillatorische Bewegung eines schnellen z-Scanners und mindestens eines schnellen Lateralscanners erzeugt, so werden Fokuswirkbereiche immer nur in einer halben Periode der oszillatorischen Bewegung gesetzt. Ist die der schnellen Scanbewegung überlagerte langsame Scanbewegung konstant, so führt dies zu regelmäßig angeordneten Strokes. Ist die Lateralkomponente der langsamen Scanbewegung identisch mit der Richtung der oszillatorischen Bewegung des mindestens einen Lateralscanners, so weisen die Strokes stets denselben Abstand zueinander auf.

Bei Nutzung der Abwärtsbewegung ist es vorteilhaft, wenn der Neigungswinkel der Strokes zur Strahlachse größer als der Fokussierwinkel der fokussierten Strahlung ist, damit ein bereits realisierter Fokuswirkbereich auch nicht auf dem Kegelmantel des Fokuskegels eines noch zu realisierenden Fokuswirkbereichs angeordnet ist.

In einer anderen Variante einer erfindungsgemäßen Anordnung ist die Steuereinrichtung derart kodiert, dass die Ausbildung der Strokes durch eine Aneinanderreihung von Fokuswirkbereichen der fokussierten Strahlung abwechselnd in einer Aufwärtsbewegung und in einer Abwärtsbewegung entlang der Scanlinie realisiert ist. Dabei entstehen Strokes mit im Wesentlichen zwei unterschiedlichen Neigungswinkeln, jedoch können hier vorteilhafterweise während der gesamten Periode einer oszillatorischen Bewegung Fokuswirkbereiche realisiert werden.

In einer Ausführungsform der erfindungsgemäßen Anordnung umfasst die Einrichtung zur Erzeugung einer Strahlung einen gepulsten Laser mit einer Laserpulsrepetitionsrate. Ein Abstand eines Fokuswirkbereichs von einem vorangehenden Fokuswirkbereich ist dann durch die Laserpulsrepetitionsrate und eine Gesamtscangeschwindigkeit, die sich aus den Scangeschwindigkeiten der langsamen und schnellen Scanbewegungen zusammensetzt, bestimmt.

Dabei sind die Scangeschwindigkeiten nicht notwendigerweise konstant. Insbesondere für die oszillatorische Bewegung gilt, dass die Geschwindigkeit am höchsten für Nulldurchgang der Oszillation und am niedrigsten am Umkehrpunkt, also beim Minimalwert und beim Maximalwert der oszillatorischen Bewegung, sind.

Von besonderen Vorteil ist es dann, wenn die Steuereinrichtung eingerichtet ist, einen Laserpuls auszublenden, wenn dessen Fokuswirkbereich einen Mindestabstand zum vorangehenden Fokuswirkbereich unterschreitet. Damit können Schäden im zur bearbeitenden transparenten Material vermieden werden, die dadurch entstehen können, dass mehrere Fokuswirkbereiche an derselben Ort oder an dicht nebeneinander befindlichen Orten im Bearbeitungsvolumen realisiert werden.

In dieser Ausführungsform der erfindungsgemäßen Anordnung werden also mehrere vorteilhafte Merkmale vereint, die sich hierbei gegenseitig positiv beeinflussen, um eine Bearbeitungsfläche präzise und in hoher Qualität mit einer fokussierten Strahlung zu bearbeiten, ohne Schäden zu verursachen.

Durch die Aneinanderreihung von Strokes, die einen Neigungswinkel zur optischen Achse aufweisen, der einerseits größer als der Fokussierwinkel ist, also so groß ist, dass bestehende Fokuswirkbereiche aus dem bereits realisierten Teil des Scanmusters von der fokussierten Strahlung nur in Bereichen hinter dem Fokuspunkt der fokussierten Strahlung durchdrungen werden, gibt es keine Abschattungseffekte. Wenn die Strokes andererseits einen Neigungswinkel aufweisen, der kleiner als 90° ist, so lassen sich damit Flächenformen erzeugen, die keine Grenzbereiche, oder für den Spezialfall der geschlossenen Flächen, maximal einen Grenzbereich zu bereits realisierten Fokuswirkbereichen aufweisen. Durch die Ausblendung von Laserpulsen in Abhängigkeit von der Geschwindigkeit der durch die schnelle und die langsame Scanbewegung zusammengesetzte Gesamtscanbewegung werden Schäden im zu bearbeitenden transparenten Material, insbesondere Schäden in einem Augengewebe, vermieden. Zudem führt eine Bearbeitung einer Fläche mit einem solchen Scanmuster zu einer effizienten Befüllung der Bearbeitungsfläche. Im Fall einer Schnittfläche, die durch die Fokuswirkbereiche eines gepulsten Lasers, insbesondere eines Femtosekunden-Lasers erzeugt wird, führt dies zu einer sauberen Schnittfläche, bei der das Risiko von Ausrissen minimiert wird.

Eine erfindungsgemäße Anordnung erlaubt also die effektive, zeitsparende Anordnung von Fokuswirkbereichen, insbesondere von Laserfokus-Wirkbereichen, in einem Scanmuster, das die Eigenschaften der Einrichtung zur Veränderung der Lage des Fokus, insbesondere eines hierin enthaltenen Scansystems, nur einen Teilbereich des dreidimensionalen Bearbeitungsvolumens schnell zugänglich zu haben, diesen Teilbereich aber langsam durch das gesamte Bearbeitungsvolumen bewegen zu können, berücksichtigt. Letzteres ermöglicht die Verwendung einer preiswerten Fokussieroptik mit kleinem Feld und der beschränkten Weite einer schnellen Fokustiefenverstellung.

Ist aufgrund einer nur geringen Ausdehnung der zu bearbeitenden Fläche eine kleine, kostengünstige Optik ausreichend, ohne dass diese dann noch verschoben werden muss, oder ist die Geschwindigkeit der Bearbeitung auch bei ausgedehnten zu bearbeitenden Flächen nicht von Belang, dann wird die oben gestellte Aufgabe auch gelöst von einer Anordnung zur Bearbeitung einer Fläche in einem Bearbeitungsvolumen eines transparenten Materials mittels einer fokussierten Strahlung, insbesondere von einer ophthalmologischen Therapievorrichtung, die eine Einrichtung zur Erzeugung einer Strahlung und eine Optik zur Fokussierung der Strahlung in einem Fokus im Bearbeitungsvolumen umfasst, wobei der Fokus der fokussierten Strahlung einen Fokussierwinkel und die fokussierte Strahlung eine Strahlachse entsprechend oben gegebener Definitionen aufweist.

Die Einrichtung zur Erzeugung einer fokussierbaren Strahlung kann wiederum einen Laser umfassen, bevorzugt einen gepulsten Laser und insbesondere einen Kurzpulslaser wie einen Pikosekunden- oder Femtosekunden-Laser mit den oben beschriebenen Eigenschaften.

Die Anordnung umfasst weiterhin eine Einrichtung zur Veränderung der Lage des Fokus der so fokussierten Strahlung mittels einer Scanbewegung in beliebiger änderbarer Richtung im Bearbeitungsvolumen, wobei das Bearbeitungsvolumen und damit auch die Scanbewegung und ein hieraus entstehende Scanmuster durch drei Raumrichtungen x, y und z beschreibbar ist. Dabei verläuft die x-Richtung und die y-Richtung nicht parallel zueinander und jeweils senkrecht zu einer Grundstellungs-Strahlachse und die z-Richtung parallel zur Grundstellungs-Strahlachse, die auch hier die Strahlachse der fokussierten Strahlung ohne eine Auslenkung des Fokus durch die Einrichtung zur Veränderung der Lage des Fokus im Bearbeitungsvolumen in den beiden Lateralrichtungen x und y kennzeichnet. Die Veränderung der Lage des Fokus erfolgt bevorzugt kontinuierlich, jedoch nicht notwendigerweise mit einer konstanten Geschwindigkeit. Hierzu kann die Einrichtung zur Veränderung der Lage des Fokus drei voneinander unabhängige Scanner, beispielsweise einen x-Scanner, einen y-Scanner und einen z-Scanner, enthalten. Diese können bezüglich erreichbarem Bearbeitungsvolumen und Geschwindigkeiten ebenso durch die oben genannten Werte charakterisiert sein. Auch kann der langsame x-Scanner kann mit dem langsamen y-Scanner in einer x-y-Scaneinheit zusammengefasst sein.

Die Anordnung ist gekennzeichnet durch eine Steuereinrichtung, die eingerichtet ist zur Steuerung der Anordnung, insbesondere zur Steuerung der Einrichtung zur Erzeugung einer Strahlung, der Einrichtung zur Veränderung der Lage des Fokus der fokussierten Strahlung und/oder der Optik. Sie ist hierzu über entsprechende Kommunikationswege mit der Einrichtung zur Erzeugung einer Strahlung, der Einrichtung zur Veränderung der Lage des Fokus der fokussierten Strahlung und/oder der Optik verbunden. Die Steuereinrichtung kann wiederum einteilig oder mehrteilig ausgeführt sein.

Erfindungsgemäß ist in die Steuereinrichtung wiederum ein Scanmuster kodiert, das durch die Scanbewegung entlang einer Scanlinie realisierbar ist, wobei die Scanbewegung mindestens eine laterale Grundkomponente in x- und/oder y-Richtung aufweist. Die Scanbewegung kann des Weiteren auch eine Grundkomponente in z-Richtung aufweisen.

Die Scanbewegung ist jedoch nicht gekennzeichnet durch eine gleichmäßige Bewegung in eine beliebige Richtung: Vielmehr ist eine laterale Grundkomponente in x- und/oder y-Richtung und ggf. auch eine Grundkomponente in z-Richtung überlagert von Komponenten synchroner Richtungswechselbewegungen in z-Richtung und in mindestens einer der lateralen Raumrichtungen x und/oder y. Die synchronen Richtungswechselbewegungen sind also Bewegungsanteile einer Gesamtbewegung in einer entsprechenden Richtung, die sich aus der jeweiligen Grundkomponente und den jeweiligen Richtungswechselbewegungen in dieser Richtung zusammensetzen.

Dabei erfolgen die synchronen Richtungswechselbewegungen in z-Richtung und in mindestens einer der lateralen Raumrichtungen x und/oder y in einer definierten zeitlichen Beziehung zueinander. Insbesondere können die Richtungswechsel gleichzeitig erfolgen. Alternativ ist aber auch ein definierter zeitlicher Versatz der synchronen Richtungswechsel möglich.

Die synchronen Richtungswechselbewegungen in z-Richtung und in mindestens einer der lateralen Raumrichtungen x und/oder y können dabei periodisch erfolgen.

Die laterale Grundkomponente in x- und/oder y-Richtung ist überlagert von synchronen Richtungswechselbewegungen in z-Richtung und in x-Richtung und/oder y-Richtung, die so aufeinander synchronisiert sind, dass bei einer positiven lateralen Grundkomponente der Scanbewegung in x- und/oder y-Richtung die Richtungswechselbewegungen in x- und/oder y-Richtung gegenläufig zu den Richtungswechselbewegungen in z-Richtung sind, und dass bei einer negativen lateralen Grundkomponente der Scanbewegung in x- und/oder y-Richtung die periodischen Richtungswechselbewegungen in x- und/oder y-Richtung gleichläufig zu den periodischen Richtungswechselbewegungen in z-Richtung sind.

Eine gleichgerichtete laterale Grundkomponente in x- und/oder y-Richtung und ggf. auch eine gleichgerichtete Grundkomponente in z-Richtung ist also überlagert von Richtungswechselbewegungen in z-Richtung zwischen einem minimalen z-Umkehrpunkt und einem maximalen z-Umkehrpunkt, die synchronisiert sind zu Richtungswechselbewegungen in mindestens einer lateralen Raumrichtung x und/oder y zwischen einem minimalen x-Umkehrpunkt und einem maximalen x-Umkehrpunkt und/oder zwischen einem minimalen y-Umkehrpunkt und einem maximalen y-Umkehrpunkt. Der Wert des jeweiligen x-, y- bzw. z-Umkehrpunkts ist dabei in der Regel keine Konstante, sondern kann beliebig innerhalb der Möglichkeiten des Bearbeitungsvolumens variieren.

Dabei sind mindestens zwei Richtungswechselbewegungen derart zueinander synchronisiert, dass der maximale x-Umkehrpunkt und/oder der maximale y-Umkehrpunkt bei einem minimalen z-Umkehrpunkt und der minimale x-Umkehrpunkt und/oder der minimale y-Umkehrpunkt bei einem maximalen z-Umkehrpunkt für eine laterale Grundkomponente in positive x- und/oder y-Richtung, sowie der minimale x-Umkehrpunkt und/oder der minimale y-Umkehrpunkt bei einem minimalen z-Umkehrpunkt und der maximale x-Umkehrpunkt und/oder der maximale y-Umkehrpunkt bei einem maximalen z-Umkehrpunkt für eine laterale Grundkomponente in negative x- und/oder y-Richtung auftritt.

Die daraus resultierende Scanbewegung ist jedoch nicht das Ergebnis des Zusammenwirkens mehrerer Scanbewegungen, die mit unterschiedlichen Scansystemen bzw. mit unterschiedlichen Scannern erzeugt werden, sondern sie wird durch ein Scansystem, beispielsweise unter Nutzung eines Scanners für jede der Raumrichtungen x, y und z, realisiert, die Teil der Einrichtung zur Veränderung der Lage des Fokus im Bearbeitungsvolumen sind.

Gibt es also eine gleichgerichtete laterale Grundkomponente in dieselbe Richtung, in der Richtungswechselbewegungen erfolgt, und liegen zudem Richtungswechselbewegungen in z-Richtung vor, so muss bei der Synchronisation der Richtungswechselbewegungen untereinander auf die genannten Bedingungen geachtet werden: Dies ermöglicht es, ein Scanmuster so zu realisieren, dass Fokuswirkbereiche auf geneigten Scanlinien entstehen, bei deren Erzeugung möglichst wenig Abschattungseffekte durch Strukturen der bereits bearbeiteten Bereiche der Bearbeitungsfläche wirksam sind.

Ein solches Arbeiten, das in jedem Moment sehr lokal beschränkt ist, ist beispielsweise in der Augenchirurgie günstig, um die Auswirkungen einer möglichen Bewegung des Auges während des Eingriffs gering zu halten. Im Gegensatz zum Stand der Technik wird mit der erfindungsgemäßen Anordnung - wie auch in einem entsprechenden Verfahren - nicht etwa über einen langen Zeitraum das gesamte Bearbeitungsvolumen bearbeitet und immer wieder nur ein kleiner Teil einer Schnittebene fertiggestellt, was sonst bei Bewegungen des Auges zu einem Versatz innerhalb einer Schnittebene führt, die dann eventuell gar nicht vollständig ausgeführt ist. Mit der erfindungsgemäßen Anordnung und dem entsprechenden Verfahren wird hingegen der Schnitt lokal sofort fertiggestellt.

Durch entsprechende Wahl der gleichgerichteten lateralen Grundkomponente in x- und/oder y-Richtung sowie der Geschwindigkeit und der "Amplitude" der Richtungswechselbewegungen in die jeweilige laterale Raumrichtung x und/oder y sowie deren Umsetzung durch Kodierung in die Steuereinrichtung ist in einer bevorzugten erfindungsgemäßen Anordnung ein Scanmuster kodiert, das eine Abfolge von Fokuswirkbereichen der fokussierten Strahlung entlang einer Scanlinie aufweist, derart dass bereits realisierte Fokuswirkbereiche stets außerhalb eines Fokuskegels, der durch den Fokus der fokussierten Strahlung und den Fokussierwinkel gebildet ist, für noch zu realisierende Fokuswirkbereiche angeordnet sind, da hiermit die Neigung der Scanlinien zur Strahlachse bestimmt werden kann.

Sehr vorteilhaft ist eine Anordnung, in dessen Steuereinrichtung ein Scanmuster kodiert ist, das zueinander benachbarte Strokes mit Neigungswinkeln zur Strahlachse aufweist, wobei ein Stroke einen gleichgerichteten Teil der Scanlinie umfasst und durch eine Aneinanderreihung von Fokuswirkbereichen der fokussierten Strahlung realisiert ist, und wobei die Neigungswinkel des Strokes größer oder gleich dem Fokussierwinkel der fokussierten Strahlung sind. Mit einem solchen Scanmuster wird eine Abfolge der Anordnung der Fokuswirkbereiche derart erreicht, dass bereits realisierte Fokuswirkbereiche stets außerhalb eines Fokuskegels, der durch den Fokus der fokussierten Strahlung und den Fokussierwinkel gebildet ist, für noch zu realisierende Fokuswirkbereiche angeordnet sind.

Für eine Anordnung von Strokes mit einem Neigungswinkel zur Strahlachse wird ein folgendes Stroke also jeweils über dem benachbarten vorangegangenem Stroke realisiert, wobei "über" eine Anordnung in einer Richtung entgegen der Strahlrichtung meint.

Die Steuereinrichtung kann dabei so kodiert sein, dass die Ausbildung der Strokes durch eine Aneinanderreihung von Fokuswirkbereichen der fokussierten Strahlung stets in einer Aufwärtsbewegung oder stets in einer Abwärtsbewegung oder abwechselnd in einer Aufwärtsbewegung und in einer Abwärtsbewegung entlang der Scanlinie realisiert ist.

Auch kann die Einrichtung zur Erzeugung einer Strahlung der erfindungsgemäßen Anordnung einen gepulsten Laser mit einer Laserpulsrepetitionsrate umfassen, und ein Abstand eines Fokuswirkbereichs von einem vorangehenden Fokuswirkbereich durch die Laserpulsrepetitionsrate und eine Scangeschwindigkeit bestimmt sein. Dann ist es vorteilhaft, wenn die Steuereinrichtung dazu eingerichtet ist, einen Laserpuls auszublenden, wenn dessen Fokuswirkbereich einen Mindestabstand zum vorangehenden Fokuswirkbereich unterschreitet.

Eine Fläche in höchster Qualität zu bearbeiten erfordert auch die Reduzierung oder im Idealfall die vollständige Vermeidung von Schädigungen des zu bearbeitenden transparenten Materials. Eine Anordnung zur Bearbeitung einer Fläche in einem Bearbeitungsvolumen eines transparenten Materials mittels einer fokussierten Strahlung, insbesondere eine ophthalmologische Therapieanordnung, enthält eine Einrichtung zur Erzeugung einer Strahlung, die einen gepulsten Laser mit einer Laserpulsrepetitionsrate umfasst, und eine Optik zur Fokussierung der Strahlung in einem Fokus im Bearbeitungsvolumen, eine Einrichtung zur Veränderung der Lage des Fokus der so fokussierten Strahlung im Bearbeitungsvolumen sowie eine Steuereinrichtung, eingerichtet zur Steuerung der Anordnung.

Auch hier kann die Einrichtung zur Erzeugung einer Strahlung einen gepulsten Laser, insbesondere einen Kurzpulslaser wie beispielsweise einen Pikosekunden- oder Femtosekunden-Laser mit den oben beschriebenen Eigenschaften umfassen. Die Einrichtung zur Veränderung der Lage des Fokus kann wiederum ein Scansystem mit einen x-Scanner, einem y-Scanner und einem z-Scanner enthalten. Diese können bezüglich erreichbarem Bearbeitungsvolumen und Geschwindigkeiten durch die oben genannten Werte charakterisiert sein.

In einer solchen Einrichtung wird ein Abstand eines Fokuswirkbereichs von einem vorangehenden Fokuswirkbereich durch die Laserpulsrepetitionsrate und eine Scangeschwindigkeit, die örtlich und zeitlich variieren kann, bestimmt. Dabei ist die Steuereinrichtung einer erfindungsgemäßen Anordnung zur Vermeidung von Schädigungen des zu bearbeitenden transparenten Materials eingerichtet, einen Laserpuls auszublenden, wenn ein Mindestabstand des Fokuswirkbereiches des Laserpulses zum vorangehenden Fokuswirkbereich unterschritten ist.

Dies ist insbesondere bei oszillierenden Scanbewegungen der Fall, bei denen es jeweils Umkehrpunkte gibt, an denen die Geschwindigkeit der entsprechenden Scanbewegung in die betreffende Richtung gegen null geht. So ist es beispielsweise sinnvoll, bei Oszillationen in z-Richtung die Laserpulse in einen Bereich um den oberen und um den unteren Umkehrpunkt auszublenden und somit eng aneinander liegende Fokuswirkbereiche oder gar mehrere Fokuswirkbereiche an identischem Ort zu vermeiden.

Die oben genannte Aufgabe wird des Weiteren auch gelöst durch ein Steuerprogrammprodukt, also ein Computerprogrammprodukt, dass zur Steuerung einer Anordnung eingesetzt werden kann.

Das erfindungsgemäße Steuerprogrammprodukt ist eingerichtet ist zur Kodierung einer Steuereinrichtung einer Anordnung zur Bearbeitung einer Fläche in einem Bearbeitungsvolumen eines transparenten Materials mittels einer fokussierten Strahlung nach einem der Ansprüche 1 bis 16.

Insbesondere gibt in einer Ausführungsvariante ein solches Steuerprogrammprodukt eine Kodierung eines Scanmusters derart vor, dass es durch schnelle Scanbewegungen in einem Teilbereich des Bearbeitungsvolumens und langsame Scanbewegungen im Beobachtungsvolumen in jeder beliebigen, durch drei Raumrichtungen bestimmten Richtung zusammengesetzt ist, wobei der Teilbereich der schnellen Scanbewegung durch die langsame Scanbewegung im gesamten Bearbeitungsvolumen bewegbar ist.

Zur Realisierung der schnellen Scanbewegung kann ein Scanmuster in einer bevorzugten Ausführungsform des erfindungsgemäßen Steuerprogrammprodukts derart kodiert sein, dass synchrone Richtungswechselbewegungen der schnellen Scanbewegung in mindestens zwei Raumrichtungen stattfinden, insbesondere, dass eine schnellen Scanbewegung durch synchrone oszillatorische Bewegungen mindestens zweier schneller Scanner erzeugt wird, wobei synchrone oszillatorische Bewegungen des schnellen z-Scanners und mindestens eines schnellen Lateralscanners vorteilhaft sind.

Vorteilhaft ist ein Steuerprogrammprodukt zur Kodierung einer schnellen Scanbewegung durch die synchronisierten oszillatorischen Bewegungen des schnellen z-Scanners zwischen einem minimalen z-Wert und einem maximalen z-Wert und eines schnellen Lateralscanners oder zweier schneller Lateralscanner in Form eines schnellen x- und/oder schnellen y-Scanners, die zwischen einem minimalen x- Wert und einem maximalen x- Wert und/oder einem minimalen y-Wert und einem maximalen y-Wert oszillieren. Die mindestens zwei oszillatorischen Bewegungen sind dabei derart zueinander synchronisiert, dass der maximale x-Wert des des schnellen x-Scanners, und/oder der maximale y-Wert des schnellen y-Scanners, bei einem minimalen z-Wert des schnellen z-Scanners sowie der minimale x- Wert des schnellen x-Scanners und/oder der minimale y-Wert des schnellen y-Scanners bei einem maximalen z-Wert des schnellen z-Scanners für eine Lateralkomponente der langsamen Scanbewegung in positive x- und/oder y-Richtung, und der minimale x- Wert des schnellen x-Scanners und/oder der minimale y-Wert des schnellen y-Scanners bei einem minimalen z-Wert des schnellen z-Scanners sowie der maximale x- Wert des schnellen x-Scanners und der maximale y- Wert des schnellen y-Scanners bei einem maximalen z-Wert des schnellen z-Scanners für eine Lateralkomponente der langsamen Scanbewegung in negative x- und/oder y-Richtung erreicht wird.

Zur Realisierung der Scanbewegung kann ein Scanmuster in einer allgemeineren, bevorzugten Ausführungsform des erfindungsgemäßen Steuerprogrammprodukts derart kodiert sein, dass eine Scanbewegung mit einer lateralen Grundkomponente in x-Richtung und/oder in y-Richtung, ggf. auch mit einer zusätzlichen Grundkomponente in z-Richtung, die eine Grundbewegung bestimmt, die beispielsweise eine gleichgerichtete Bewegung vorgibt, überlagert ist mit synchronen Richtungswechselbewegungen in z-Richtung und in x-Richtung und/oder y-Richtung, die so aufeinander synchronisiert werden, dass bei einer positiven lateralen Grundkomponente der Scanbewegung in x- und/oder y-Richtung die Richtungswechselbewegungen in x- und/oder y-Richtung gegenläufig zu den Richtungswechselbewegungen in z-Richtung erfolgen, und dass bei einer negativen lateralen Grundkomponente der Scanbewegung in x- und/oder y-Richtung die Richtungswechselbewegungen in x- und/oder y-Richtung gleichläufig zu den Richtungswechselbewegungen in z-Richtung erfolgen.

Bevorzugt ist in einem erfindungsgemäßen Steuerprogrammprodukt ein Scanmuster so kodiert, das es eine Abfolge von Fokuswirkbereichen der fokussierten Strahlung entlang einer Scanlinie aufweist, derart dass bereits realisierte Fokuswirkbereiche stets außerhalb eines Fokuskegels, der durch den Fokus der fokussierten Strahlung und dem Fokussierwinkel gebildet ist, für noch zu realisierende Fokuswirkbereiche angeordnet sind.

Ein besonders bevorzugtes erfindungsgemäßes Steuerprogrammprodukt enthält die Kodierung eines Scanmusters mit zueinander benachbarten Strokes mit Neigungswinkeln zu einer Strahlachse der fokussierten Strahlung, wobei ein Stroke einen gleichgerichteten Teil einer Scanlinie umfasst, und durch eine Aneinanderreihung von Fokuswirkbereichen der fokussierten Strahlung realisiert ist, und wobei die Neigungswinkel der Strokes zur Strahlachse größer oder gleich dem Fokussierwinkel der fokussierten Strahlung sind.

Ein Scanmuster kann des Weiteren so durch ein Steuerprogrammprodukt kodiert werden, dass die Ausbildung eines Strokes durch eine Aneinanderreihung von Fokuswirkbereichen der fokussierten Strahlung stets in einer Aufwärtsbewegung oder stets in einer Abwärtsbewegung entlang der Scanlinie realisiert ist, oder dass die Ausbildung der Strokes durch eine Aneinanderreihung von Fokuswirkbereichen der fokussierten Strahlung abwechselnd in Aufwärtsbewegung und in Abwärtsbewegung realisiert ist.

Eine Ausführungsform des erfindungsgemäßen Steuerprogrammprodukts für eine Anordnung, die eine Einrichtung zur Erzeugung einer Strahlung in Form einer gepulsten Laserstrahlung mit einer Laserpulsrepetitionsrate umfasst, enthält eine Kodierung eines Scanmusters derart, dass ein Laserpuls ausgeblendet ist, wenn dessen Fokuswirkbereich einen Mindestabstand zum vorangehenden Fokuswirkbereich unterschreitet.

Die vorliegende Erfindung soll nun anhand von Ausführungsbeispielen erläutert werden. Es zeigt:
- die Fig. 1a und 1b: die Realisierung zweier Schnittflächen in einem Bearbeitungsvolumen eines transparenten Materials mit einer optischen Strahlung nach dem Stand der Technik wie oben beschrieben.
- die Fig. 2: die Realisierung einer Schnittfläche in einem Bearbeitungsvolumen eines transparenten Materials durch laterales Wobbeln wie oben beschrieben.
- die Fig. 3: ein erstes Beispiel einer Anordnung zur Bearbeitung einer Fläche in einem Bearbeitungsvolumen eines transparenten Materials mittels einer fokussierten Strahlung.
- die Fig. 4: ein Schema des optischen Gesamtaufbaus einer erfindungsgemäßen ophthalmologischen Therapieanordnung mit einer Optik zur Fokussierung einer Strahlung und einer Einrichtung zur Veränderung der Lage des Fokus der Strahlung.
- die Fig. 5: ein z-Scan-Modul aus schnellem und langsamen z-Scanner.
- die Fig. 6: die Divergenzänderung bei Bewegung des Fokus in z-Richtung.
- die Fig. 7: den Aufbau eines Scanmusters für eine zu bearbeitende Fläche in einem Bearbeitungsvolumen.
- die Fig. 8a bis 8c: die Lage des Fokuskegels eines zu realisierenden Fokuswirkbereiches zu bereits realisierten Fokuswirkbereichen für verschiedene Scanmuster.
- die Fig. 9: die vorteilhafte Aneinanderreihung geneigter Scanlinien zur Bearbeitung einer in sich geschlossenen, zylinderförmigen Fläche.
- die Fig. 10: die Überlagerung einer Sinus-Schwingung des schnellen z-Scanners mit einer synchronen Sinus-Schwingung eines schnellen lateralen Scanners mit langsamer Veränderung des Schwingungsmittelpunktes in positive x-Richtung zur Erzeugung gleichgerichteter Strokes.
- die Fig. 11: die Überlagerung einer Sinus-Schwingung des schnellen z-Scanners mit einer synchronen Sinus-Schwingung eines schnellen lateralen Scanners mit langsamer Veränderung des Schwingungsmittelpunktes in negative x-Richtung zur bidirektionalen Erzeugung von Strokes.
- die Fig. 12: ein erstes Scanmuster zu Generierung eines Capsulotomieschnittes.
- die Fig. 13: ein zweites Scanmuster zu Generierung eines Capsulotomieschnittes.
- die Fig. 14: ein drittes Scanmuster zu Generierung eines Capsulotomieschnittes.
- die Fig. 15a bis 15c: verschiedene Phasen eines Scanmusters zur Generierung eines Linsenfragmentationsschnittes, die Fig. 12d und 12e eine vorteilhaftes Scanmuster bei gekreuzten Schnittebenen.
- die Fig. 16a bis 16c: ein Scanmuster zur Generierung einer Arcuate Incision in verschiedenen Ansichten.
- die Fig. 17a bis 17c: ein erstes Scanmuster zu Generierung einer Access Incision in verschiedenen Phasen.
- die Fig. 18a bis 18c: ein zweites Scanmuster zu Generierung einer Access Incision in verschiedenen Phasen.
- die Fig. 19: Eine zweites Beispiel einer Anordnung zur Bearbeitung einer Fläche in einem Bearbeitungsvolumen eines transparenten Materials mittels einer fokussierten Strahlung.

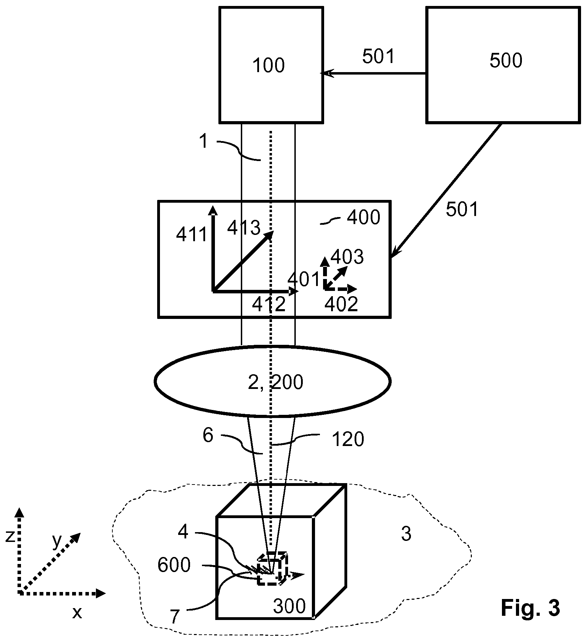

Die Fig. 3 zeigt eine erstes Beispiel einer ophthalmologischen Therapieanordnung zur Bearbeitung einer Fläche in einem Bearbeitungsvolumen eines transparenten Materials mittels einer fokussierten Strahlung.

Die Anordnung enthält eine Einrichtung zur Erzeugung einer Strahlung 100, die einen Femtosekunden-Laser mit einer Wellenlänge im Bereich von 1020 - 1060nm umfasst. Die Pulsdauer dieses Femtosekunden-Lasers beträgt 500-600fs , die Pulsenergie ca. 10µJ und die Laserpulsrepetitionsrate ca. 100kHz .

Des Weiteren enthält die Anordnung eine Optik 2, 200 zur Fokussierung der Strahlung in einem Fokus 4, mit einer numerischen Apertur von 0,2, die eine Bildfeldgröße von ca. 1mm aufweist.

Auch enthält die Anordnung eine Einrichtung zur Veränderung der Lage der fokussierten Strahlung. Diese kann in jeder beliebigen, durch die drei Raumrichtungen bestimmten Richtung eine langsame Scanbewegung im Bearbeitungsvolumen 300 des transparenten Materials 3, hier also des Auges, und eine von der langsamen Scanbewegung unabhängige schnelle Scanbewegung in einem Teilbereich 600 des Bearbeitungsvolumens 300 vollziehen, wobei der Teilbereich 600 der schnellen Scanbewegung durch die langsame Scanbewegung im gesamten Bearbeitungsvolumen 300 bewegbar ist.

Es werden insgesamt sechs Scanner eingesetzt. Für jede der Raumrichtung x, y und z, wobei z-Richtung parallel und die x- und y-Richtung senkrecht der Grundstellungs-Strahlachse 120 verläuft, gibt es jeweils einen schnellen Scanner 401, 402, 403, der es erlaubt, einen Teilbereich 600 von ca. 1mm x 1mm x 1mm des dreidimensionalen Bearbeitungsvolumen 300 mit mehreren hundert Hertz zu scannen. Zusätzlich gibt es für jede Raumrichtung einen langsamen Scanner 411, 412, 413. Diese erlauben es, das für die o.g. Aufgaben der Generierung von Schnitten in einem Augengewebe 3 komplette nötige Bearbeitungsvolumen 600 von ca. 15mm x 15mm x 15mm zu erreichen.

Die insgesamt vollzogene Scanbewegung zu einem Zeitpunkt t setzt sich aus langsam veränderlichen Komponenten in x-, y- und z-Richtung, die mit dem Index s gekennzeichnet sind, und schnell veränderlichen Komponenten in x-, y- und z-Richtung, die mit dem Index f gekennzeichnet sind, zusammen: $\left(\begin{matrix}x \left(t\right) \\ y \left(t\right) \\ z \left(t\right)\end{matrix}\right) = \left(\begin{matrix}{x}_{s} \left(t\right) \\ {y}_{s} \left(t\right) \\ {z}_{s} \left(t\right)\end{matrix}\right) + \left(\begin{matrix}{x}_{f} \left(t\right) \\ {y}_{f} \left(t\right) \\ {z}_{f} \left(t\right)\end{matrix}\right) .$

Die langsam veränderlichen Komponenten (xₛ, yₓ, zₛ) können einen beliebigen Zeitverlauf haben und müssen nur unter einer Maximalgeschwindigkeit vₘₐₓ und/oder einer Maximalbeschleunigung aₘₐₓ bleiben. $\frac{dx \left(t\right)}{dt} \leq {v}_{max} ; \frac{{d}^{2} x \left(t\right)}{{dt}^{2}} \leq {a}_{max}$ (dito für y(t) und z(t)).

Die schnell veränderlichen Komponenten (x_{f}, y_{f}, z_{f}) unterliegen nicht diesen Einschränkungen. Zur Flächenfüllung müssen aber wiederkehrend ähnliche Bewegungsbahnen durchlaufen, und damit die Strokes 7 erzeugt werden. Ein Beispiel für wiederkehrend ähnliche Bewegungsbahnen, das aber an dieser Stelle nicht beschränkend sein soll, wäre eine Oszillation mit einer Periodendauer T: ${x}_{f} = X \left(t\right) sin \left(2π\frac{t}{T}\right) + O \left(t\right) .$

Die Amplitude X und eine Mittenposition O dürfen sich dabei auch, ähnlich der langsamen Komponenten der Scanbewegung, langsam mit der Zeit ändern. Es ist nur erforderlich, dass sich wiederkehrend Bewegungsbahnen, also die durchlaufenen Ortskurven, ähneln. Dass diese immer in ähnlichen Zeiten durchlaufen werden, ist nicht zwingend notwendig aber ein typischer Realisierungsfall. Durch die Zusammensetzung mit der langsamen Bewegung werden dann ähnliche Strokes 7 dicht nebeneinander platziert und bilden eine Schnittfläche 11.

Die Anordnung enthält weiterhin ein einteiliges zentrales Steuersystem 500, das über die Kommunikationswege 501 mit der Einrichtung zur Erzeugung einer Strahlung 100, also dem Femtosekunden-Lasersystem, und mit der Einrichtung zur Veränderung der Lage 400 des Fokus 4 verbunden ist und das eingerichtet ist, sowohl das Femtosekunden-Lasersystem als auch alle Scanner 401, 402, 403, 411, 412, 413 der Einrichtung zur Veränderung der Lage 400 des Fokus 4 zu steuern.

In diesem Beispiel beträgt die Laserpulsrepetitionsrate 100kHz. Benachbarte Fokuswirkbereiche solle einen Abstand von ca. 10µm aufweisen. Die Scangeschwindigkeit der schnellen Scanner beträgt ca. 1000mm/s. Daraus ergeben sich bei einer Größe von ca. 1mm × 1mm × 1mm des Teilbereichs 600 des Bearbeitungsvolumens 300 für die schnellen Scanner 401, 402, 403 dann 100 Pulse in 1ms. Danach sollte auch die langsame Scanbewegung in der entsprechenden Richtung zu einem Voranschreiten von ca. 10µm geführt haben. Die langsamen Scanner 411, 412, 413 weisen damit eine Scangeschwindigkeit von ca. 10mm/s auf. Damit ergibt sich ein Verhältnis von schneller zu langsamer Scanbewegung von ca. 100:1.

Diese Anordnung wird zur Erzeugung von Schnitten mittels Photodisruption des Femtosekunden-Lasers genutzt: Mit dieser Anordnung können beispielsweise Zugangsschnitte einer lateralen Größe von 2mm, eine Capsulotomie mit einem lateralen Durchmesser von 5mm oder Relaxationsschnitte in der Hornhaut über einen lateralen Durchmesser von 11mm ausgeführt werden. In der Tiefe, also in z-Richtung kann bei einfachen Schnitten, bei denen eine gewisse Schräglage berücksichtig werden soll, eine Schnitttiefe von 500µm erzeugt werden, eine Linsendicke von 3-5mm durchdrungen oder aber anderweitige Schnitte in die Hornhaut oder die Line von 10 bis 12mm Tiefe durchgeführt werden.

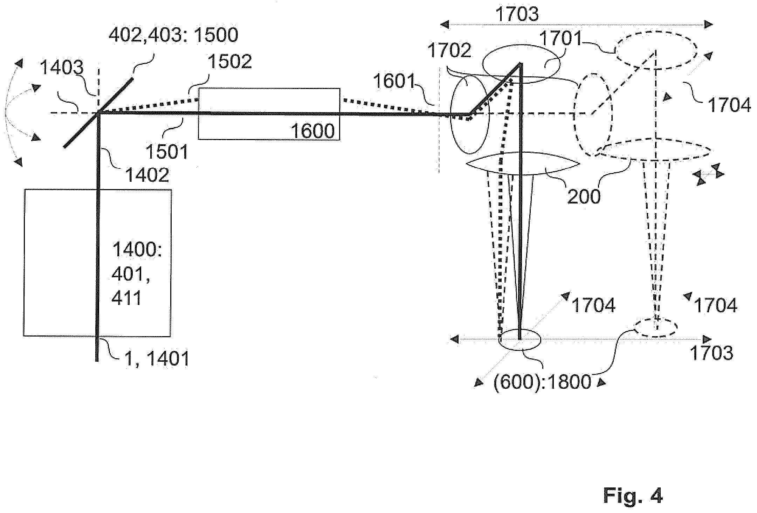

Die Fig. 4 zeigt ein Schema des optischen Gesamtaufbaus einer erfindungsgemäßen ophthalmologischen Therapieanordnung mit einer Fokussieroptik 200 zur Fokussierung einer Strahlung 1 und einer Einrichtung zur Veränderung der Lage des Fokus der Strahlung. Dieses ist beispielsweise im ersten Beispiel der ophthalmologischen Therapieanordnung der Fig. 3 einsetzbar.

Ein z-Scan-Modul 1400, 401, 411 erzeugt aus dem Eingangsstrahl 1401 mit konstanter Divergenz, konstanter Auslenkung und konstantem Durchmesser einen Strahl 1402 mit modulierter Divergenz, aber immer noch konstanter Auslenkung und konstantem Durchmesser in der Austrittspupille 1403.

Ein schneller x/y-Scanner 402, 403- ein sogenannter Teilfeldscanner 1500 -, der um zwei zueinander senkrechte Achsen schwenkbar ist und eine dabei oszillierende Bewegungen um einen Nullpunkt um beide Achsen vollziehen kann, prägt dem divergenzmodulierten Strahl 1402 zusätzlich eine laterale Auslenkung auf, die aus dem unausgelenkten, divergenzmodiulierten Strahl 1501 einen ausgelenkten divergenzmodulierten Strahl 1502 macht.

Die Pupillenebene 1403, in der der Strahldurchmesser konstant ist, wird vom Relay 1600 in die Eingangspupille 1601 der Fokussieroptik 200 abgebildet, so dass der Strahl dort auch einen konstanten Durchmesser aber eine Divergenz- und Auslenkungsmodulation aufweist. Somit hat der fokussierte Strahl letztlich eine näherungsweise konstante numerische Apertur NA, die unabhängig von der Divergenz, also der z-Position des Fokus 4 und der Auslenkung, also der x-y-Position des Fokus 4 ist.

Das laterale Teilscanfeld 1800 des schnellen x/y-Scanners, also die x-y-Ausdehnung des Teilbereichs 600 der schnellen Scanbewegung, kann zudem noch durch eine wahlfreie laterale Verschiebung der Fokussieroptik 200 bewegt werden. Die Nachführung des Strahls mit der Bewegung der Fokussieroptik 200 wird durch die Spiegel 1701 und 1702 bewirkt, die sich jeweils entlang der Achse des auf sie eintreffenden nichtausgelenkten Strahls bewegen. Der Spiegel 1701 bewegt sich in Richtung 1704, die hier die y-Richtung sein soll, wie auch in Richtung 1703, die die x-Richtung sein soll, und der Spiegel 1702 in Richtung 1703, also die x-Richtung, welche hier senkrecht zur y-Richtung 1704 verläuft. Die Bewegung der Spiegel 1701 und 1702 ist dabei an die Bewegung der Fokussieroptik gekoppelt.

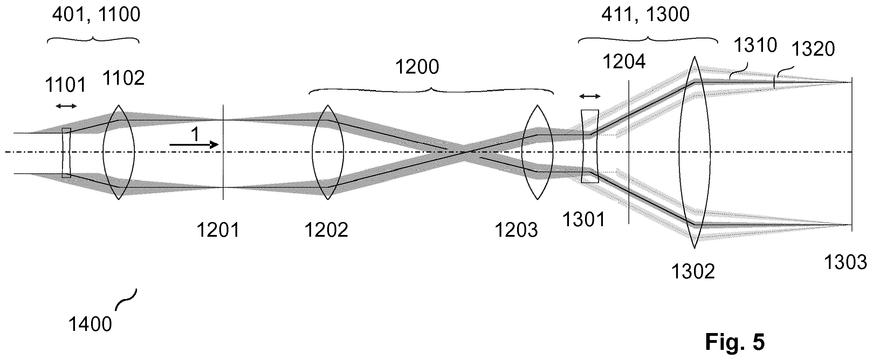

Die Fig. 5 zeigt Details eines z-Scan-Moduls 1400 das einen schnellen z-Scanner 401 und einen langsamen z-Scanner 411 enthält. Ein erstes Strahlaufweitungs-Teleskop 1100 ist dabei als schneller z-Scanner 401 ausgeführt. Es enthält eine entlang der z-Richtung bewegliche Linse 1101, die schnell um ihre Nulllage oszilliert wird. Es ist vorteilhaft eine solche schnell oszillierende Linse, wie hier gezeigt, im Strahlengang nahe der Strahlungsquelle 100 anzuordnen, da hier der Strahl noch einen kleinen Durchmesser hat, und diese bewegliche Linse 1101 an dieser Stelle als kleinstes, leichtestes Element ausgeführt werden kann. Eine negative, also konkave, Linse als bewegliche Linse 1101 des Strahlaufweitungs-Teleskops bewirkt in der Austrittspupille 1201 eine Oszillation der Strahldivergenz bei konstantem Strahldurchmesser.

Ein Relay-Teleskop 1200 bildet die Austrittspupille 1201 des schnellen z-Scanners 401 in die Eintrittspupille 1204 eines weiteren Strahlaufweitungs-Teleskops 1300 ab. Dieses weitere Strahlaufweitungs-Teleskop 1300 ist als langsamer z-Scanner 411 mit wahlfreier z-t-Kurve und weitem Scanbereich, derart, dass die gesamte Höhe z des Bearbeitungsvolumens 300 (siehe Fig. 4) überstrichen werden kann, ausgeführt. Dazu kann die Linse 1301 mit beliebigen Orts-Zeit-Kurven, jedoch geschwindigkeits- und beschleunigungslimitiert, angesteuert bewegt werden.

In der Austrittspupille 1303 dieses z-Scan-Moduls 1400 steht dann ein Strahl mit wahlfrei in einem weiten Einstellbereich 1320 langsam einstellbarer Divergenz mit festem Strahldurchmesser zur Verfügung, dessen Divergenz zudem mit fester, durch die Amplitude der Oszillation von Linse 1101 vorgegebener Amplitude in einem kleinerem Winkelbereich bzw. Einstellbereich 1310 um die mit der langsam aber wahlfrei beweglichen Linse 1301 eingestellte Nulllage oszilliert.

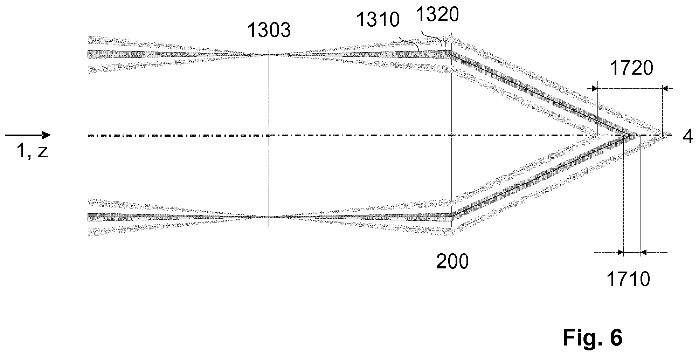

In der Fig. 6 ist die Divergenzänderung bei einer Bewegung des Fokus 4 in z-Richtung dargestellt. Die Fokussieroptik 200 überträgt die schnelle Divergenzoszillation im Winkelbereich 1310, die durch den schnellen z-Scanner hervorgerufen wird, und die langsame Divergenzvariation im Winkelbereich 1320, die durch den langsamen z-Scanner hervorgerufen wird, in eine Oszillation der z-Position des Fokus 4 im z-Bereich 1710 und Variation der Nulllage der Oszillation im z-Bereich 1720. Der Öffnungswinkel des fokussierten Strahls, also die numerische Apertur NA, bleibt konstant, wenn die Austrittspupille 1303 des langsamen z-Scanners 411 in die Eintrittspupille der Fokussieroptik 200 gelegt wird.

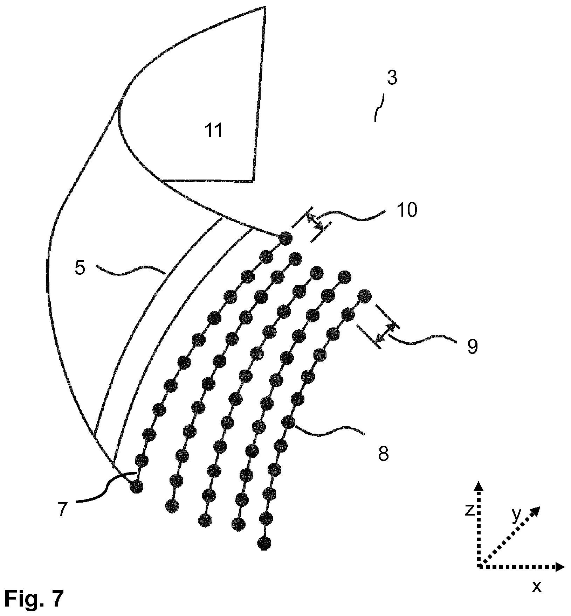

In der Fig. 7 wird der prinzipielle Aufbau eines Scanmusters für eine zu bearbeitende Fläche 11 in einem dreidimensionalen Bearbeitungsvolumen eins transparenten Materials dargestellt, wobei es sich in diesem Beispiel um eine mehrfach gewölbte Schnittfläche 11 handelt, die mit Hilfe einer gepulsten Laserstrahlung erzeugt werden soll.

Ein solches Scanmuster besteht aus einzelnen Strokes 7, die jeweils durch eine Abfolge von Laserfokuswirkbereichen 8 auf einer Scanlinie 5 realisiert werden. Die einzelnen Strokes 7 werden zu der gewünschten Schnittfläche 11 so aneinandergereiht, dass sie die Schnittfläche 11 füllen und die einzelnen Laserfokuswirkbereiche 8 nicht nur zu ihrem vorangehenden und nachfolgenden Laserfokuswirkbereich 8 auf demselben Stroke 7, sondern auch zu den Laserfokuswirkbereichen 8 der benachbarten Strokes 7 einen in etwa gleichen Abstand 9 haben, wobei Schwankungen des Abstands beispielsweise um einen Faktor 2 unproblematisch sind. Dabei bleibt jeder Stroke 7 innerhalb des momentan zugänglichen Teilbereiches 300 der Optik 2, 200, der sich aber langsam zur Ausbildung der Fläche 11 verschieben kann. Die Schnittfläche 11 hat dann eine "bandförmige" Gestalt.

Aufgrund der Flexibilität der Scanbewegungen, also die Möglichkeit zur Bewegung in eine beliebige Richtung im Bearbeitungsvolumen 300, und insbesondere bei Nutzung einer Anordnung, die eine schnelle Scanbewegung in beliebiger Richtung in einem Teilbereich 600 des Bearbeitungsvolumens 300 unabhängig von einer langsamen Scanbewegung in beliebiger Richtung im Bearbeitungsvolumen 300 ermöglicht, ist durch eine vorteilhafte Anordnung der einzelnen Strokes 7 eine beliebige Wölbung in hoher Qualität erzielbar.

Die Strokes 7 werden in diesem Beispiel durch eine synchrone oszillatorische Bewegung eines schnellen z-Scanners 401 sowie der schnellen Lateralscanner 402, 403, also eines schnellen x-Scanners 402 und eines schnellen y-Scanners 403, realisiert. Einer dieser Scanner 401, 402, 403 kann ein resonanter Scanner sein, die anderen müssen sich auf dessen Resonanzfrequenz synchronisieren lassen.

Das Scanmuster ergibt sich durch die Nebeneinanderplatzierung der Scanlinien 5 der einzelnen Strokes 7. Das wird durch eine langsame Scanbewegung mit Hilfe der langsamen Ablenksystems bzw. Scansystems, 411, 412, 413 bewirkt, das eine langsamen x-Scanner, einen langsamen y-Scanner und einen langsamen z-Scanner enthält, mit denen die Optik 2, 200 selbst bewegt wird. Durch eine Kombination von langsamer Scanbewegung und schneller oszillatorischer Bewegung wird also die Mittellage der oszillatorischen Bewegung in beliebiger, gewünschter Richtung im Bearbeitungsvolumen 300 langsam verschoben.

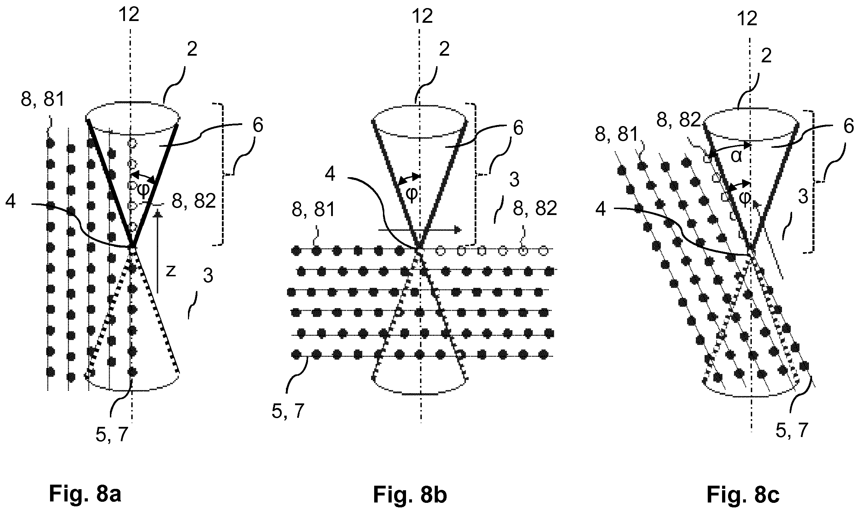

Die Fig. 8a bis 8c illustrieren die unterschiedlichen Situationen für die Lage des Fokuskegels 6 einer fokussierten Laserstrahlung für einen zu realisierenden Laserfokuswirkbereich 8, 82 zu den bereits realisierten Laserfokuswirkbereichen 8, 81 in verschiedenen Scanmustern. Unter dem Fokuskegel 6 wird dabei nur der in diesen Figuren noch einmal bildlich mit einer Klammer gekennzeichnete Teil verstanden, nicht etwa der weitere Kegel, der in Strahlrichtung hinter dem Laserfokuswirkbereich 8, 81, 82 durch das "Auseinanderlaufen" der Strahlung entsteht.

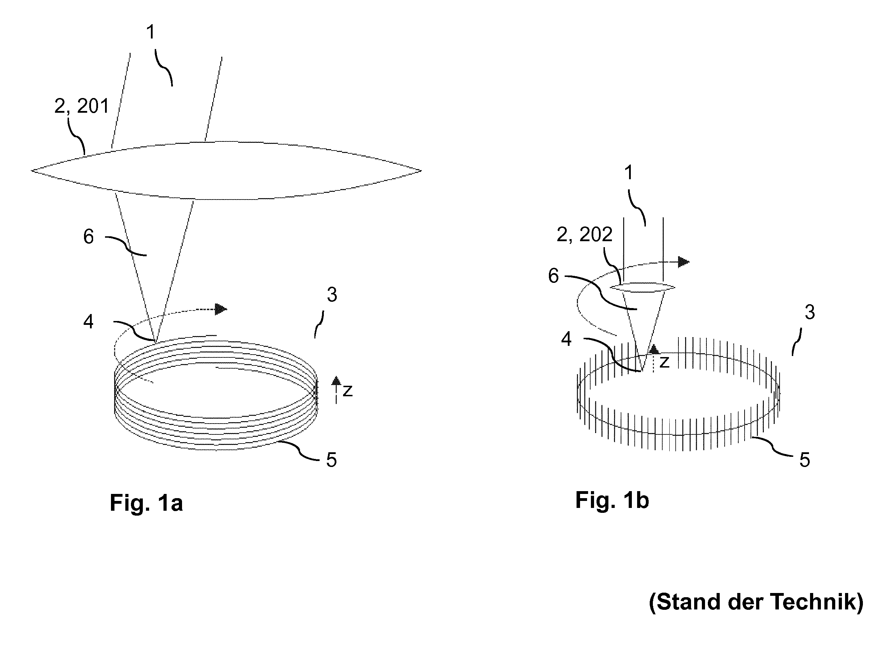

In der Fig. 8a, in der die Laserfokuswirkbereiche 8, wie im Stand der Technik und der Fig. 1b bereits gezeigt, entlang einer senkrechten Scanlinie 5 gesetzt werden, das Scanmuster also spaltenweise aufgebaut wird, ragt der Fokuskegel 6 noch zu realisierender Laserfokuswirkbereiche 8, 82 in bereits realisierte Laserfokuswirkbereiche 8, 81 der bereits realisierten Strokes 7 und führt damit zu Abschattungen.

Bereits realisierte Laserfokuswirkbereiche 8, 81 sollten sich bei der Einbringung der noch zu realisierenden Laserfokuswirkbereiche 8, 81 deshalb nicht im Fokuskegel 6 des Laserfokus 4 befinden. Beim zeilenweisen Aufbau des Scanmusters, beginnend mit der unteren Zeile wie in Fig. 8b gezeigt, ist dies stets gegeben. Allerdings tritt dann ein Abschattungsproblem durch bereits realisierte Laserfokuswirkbereiche 8, 81 zumindest in den Randbereichen einer schon erzeugten Teilfläche zu einer nächsten zu erzeugenden Teilfläche einer Bearbeitungsfläche 11 - wie oben bereits beschrieben - auf.

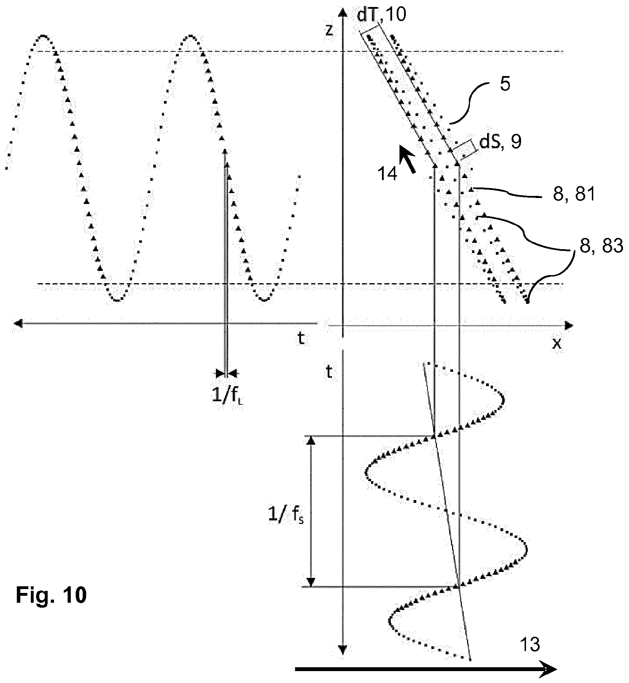

Durch Neigung der Scanlinien 5 und damit der Strokes 7 derart, dass der Neigungswinkel α der Strokes 7 zur Strahlachse 12 größer ist als der Fokussierwinkel φ, also dem Winkel zwischen einer auf dem Kegelmantel des Fokuskegels 6 verlaufenden Geraden und der Strahlachse 12, und einer solchen Abfolge der einzelnen Strokes 7, dass ein noch zu realisierendes geneigter Stroke 7 in positiver z-Richtung, also entgegen der Strahlrichtung der fokussierten Strahlung über einem bereits realisierten geneigtem Stroke 7 erzeugt wird, wie in der Fig. 8c gezeigt, wird jegliche Abschattungswirkung bereits realisierter Laserfokuswirkbereiche 8, 81 für noch zu realisierende Laserfokuswirkbereiche 8, 82 vermieden. Mit einem entsprechenden Neigungswinkel α des Strokes 7 bzw. der Scanlinie 5, auf der der Stroke 7 realisiert werden soll, ist die Platzierung des Laserfokus 4 an einen beliebigen Ort der Scanlinie 5 des zu realisierenden Strokes 7 derart möglich, dass die Scanlinie 5 den Fokuskegel 6 der Laserstrahlung nur im Fokuspunkt 4 durchdringt. Das Scanmuster der Fig. 8c kann erzeugt werden wie in der Fig. 10 detailliert dargestellt.

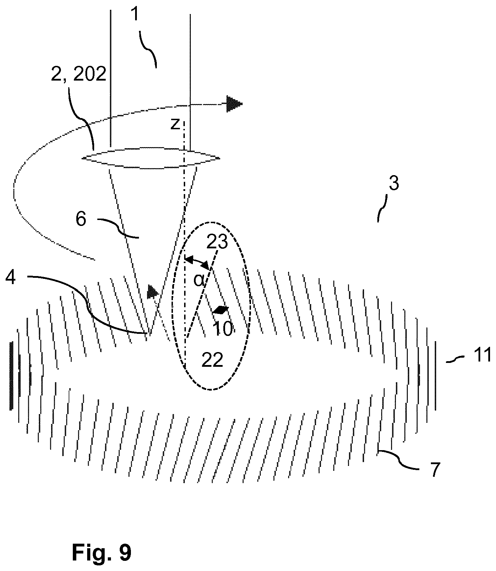

Die Fig. 9 zeigt die vorteilhafte Aneinanderreihung geneigter Strokes 7 zur Bearbeitung einer in sich geschlossenen, zylinderförmigen Fläche 11. Dabei werden dies Strokes 7 so aneinandergereiht, dass bereits bestehende Strokes 7 aus dem bereits realisiertem Teil des Scanmusters nicht den Fokuskegel 6 der Laserstrahlung, sondern nur den wieder auseinanderlaufenden weiteren Kegel in Bereichen hinter dem Laserfokus 4 durchdringen. Die Bearbeitung der Fläche 11 wird somit nicht durch Abschattungen, die von bereits bestehenden Strokes 7 erzeugt werden, behindert.

Um die Fläche 11 schließen zu können, ohne dass dabei bereits realisierte Laserfokuswirkbereiche 8, 81 in den Fokuskegel 6 des Fokus 4 für noch zu realisierende Laserfokuswirkbereiche 8, 82 geraten, sollte dabei die ersten Strokes 7 so kodiert werden, wie es im Bereich 22 der Fig. 9 dargestellt ist. Eine Linie 23 durch die Startpunkte der ersten in einem Abstand 10 nebeneinander realisierten Strokes 7 sollte mit einer Gerade in z-Richtung, die parallel der Grundstellungs-Strahlachse 12 ist, einen Winkel α bilden, der größer ist als der Fokussierwinkel φ. Um die Fläche 11 schließlich zu schließen, werden diese ersten Strokes 7 des Bereichs 22 schließlich jeweils durch einen zweiten Stroke 7 so verlängert, dass die gewünschte Gesamthöhe in z-Richtung erreicht wird.

Die geneigten Strokes 7 bzw. geneigten Scanlinien 5 mit einem Neigungswinkel α, der größer ist als der Fokussierwinkel φ, weisen trotz ihrer Neigung eine hinreichend große z-Ausdehnung zur effizienten Befüllung der Bearbeitungsfläche 11 mit Laserfokuswirkbereichen 8 auf.

Im Folgenden wird nun die Generierung einer geneigten Scanlinie 5 und geneigter Strokes 7 durch synchrone Richtungswechselbewegungen, insbesondere durch synchrone oszillatorische Bewegungen, eines schnellen z-Scanners 401 und mindestens einem der schnellen Lateralscanner 402, 403 beschrieben.

In der Fig. 10 ist zunächst die Überlagerung einer Sinus-Schwingung des schnellen z-Scanners mit einer synchronen Sinus-Schwingung eines schnellen lateralen Scanners in x-Richtung mit langsamer Veränderung des Schwingungsmittelpunktes in positive x-Richtung zur Erzeugung gleichgerichteter Strokes 7 dargestellt.

Eine Sinus-Schwingung des schnellen z-Scanners 401, die im z-t-Diagramm links oben dargestellt ist, wird mit einer synchronen Sinus-Schwingung eines schnellen x-Scanners 402 mit einer langsamer Veränderung des Schwingungsmittelpunktes, die im x-t-Diagramm rechts unten dargestellt ist, zu einem geneigten Scanmuster wie im z-x-Diagramm rechts oben dargestellt, überlagert. Alle Punkte stellen zunächst potentielle Schusspositionen eines hierfür genutzten Kurzpulslasers aufgrund von dessen Repetitionsrate, also Laserfokuswirkbereiche 8, dar. Dabei markieren die Dreiecke die tatsächlich realisierten, also nicht geblockten, Laserfokuswirkbereiche 8, 81. In diesem Fall werden Laserfokuswirkbereiche nur in der Aufwärtsbewegung 14 realisiert. Laserpulse in den Umkehrpunkten der Sinus-Bewegung und in der Abwärtsbewegung werden ausgeblendet.

Die schnellen Scanner für die z-Richtung und für die die lateralen Raumrichtungen x und/oder y führen synchrone Sinus-Schwingungen ohne Phasenverschiebung aus. Eine genau gegenphasige Schwingung kann durch eine negative Amplitude realisiert werden. Die Bahn des Laserfokus 4 beschreibt dann eine Gesamt-Scanlinie 5. Wird zusätzlich eine Bewegung vom langsamen Scansystem ausgeführt bzw. der Schwingungsmittelpunkt der Oszillation langsam verändert, bewegt sich die Scanlinie 5 während ihrer Generation durch das Bearbeitungsvolumen 300 und hinterlässt eine in das Bearbeitungsvolumen 300 "gewickelte" sinusartige Kurve: $\vec{r} \left(t\right) = \left(\begin{matrix}x \left(t\right) \\ y \left(t\right) \\ z \left(t\right)\end{matrix}\right) = \left(\begin{matrix}{A}_{x} \\ {A}_{y} \\ {A}_{z}\end{matrix}\right) sin \left(2{πf}_{S}t\right) + \left(\begin{matrix}{v}_{x} \\ {v}_{y} \\ {v}_{z}\end{matrix}\right) t + \left(\begin{matrix}{x}_{0} \\ {y}_{0} \\ {z}_{0}\end{matrix}\right)$ fₛ ist die Frequenz der Sin-Schwingung der Scanner. Die langsame Bewegung kann lokal linear approximiert werden. Die x-, y- und z-Komponenten der Momentan-Amplitude der Oszillation Aₓ, A_{y}, A_{z}, die x-, y- und z-Komponenten der Momentan-Geschwindigkeit der langsamen Scanbewegung vₓ, v_{y} ,v_{z}, und die Momentan-Position, also die Ausgangsposition im Raum, x₀, y₀ und z₀ sind im Vergleich zur Schwingungsdauer der schnellen Scanner so langsam zeitlich veränderlich, dass sie für eine Periode der Schwingung als konstant angenommen werden können.

Gibt der Laser Pulse mit einer festen Repetitionsrate ab, werden im Raum Laserfokuswirkbereiche 8 mit einem Spot-zu-Spot-Abstand, also einem Abstand der zweier Laserfokuswirkbereiche dS, 9, hinterlassen. Die Spot-zu-Spot-Abstände dS, 9 variieren mit der Momentan-Position der Sinusschwingung und sind im Nulldurchgang der Schwingung am größten und in den Umkehrpunkten fast null.

Eine gute Approximation für dS ist: $dS = \left‖\frac{d \vec{r}}{dt}\right‖ ⋅ \frac{1}{{f}_{L}}$ f_{L} ist die Laserpulsrepetitionsrate, die ein ganzzahliger Teil der Grund-Repetitionsrate des Lasers ist. Sie ist, je nach Austastverhältnis der Pulse jeder erste, jeder zweite, jeder dritte Puls, etc.

Es ist: $\frac{d \vec{r} \left(t\right)}{dt} = 2 {πf}_{S} \left(\begin{matrix}{A}_{x} \\ {A}_{y} \\ {A}_{z}\end{matrix}\right) cos \left(2{πf}_{S}t\right) + \left(\begin{matrix}{v}_{x} \\ {v}_{y} \\ {v}_{z}\end{matrix}\right)$

In den Nulldurchgängen ist dS: $\hat{dS} = \frac{2 {πf}_{S}}{{f}_{L}} \left‖\left(\begin{matrix}{A}_{x} \\ {A}_{y} \\ {A}_{z}\end{matrix}\right)\right‖ + \left‖\left(\begin{matrix}{v}_{x} \\ {v}_{y} \\ {v}_{z}\end{matrix}\right)\right‖ .$

Durch ein Ausblenden der Laserpulse, und damit der Laserfokuswirkbereiche 8, 83, in den Umkehrpunkten kann eine zu starke Variation der Spot-zu-Spot-Abstände dS, 9, und insbesondere das Realisieren von Laserfokuswirkbereichen in zu größer Nähe zueinander vermieden werden. Zum Beispiel können die Laserpulse dann ausgeblendet werden, wenn der Abstand zweier Laserfokuswirkbereiche, also der Spot-zu-Spot-Abstand dS auf zirka die Hälfte des Wertes im Nulldurchgang abgefallen ist, also wenn: $cos \left(2{πf}_{S}t\right) = \pm 1 / 2 .$

Die "Schraffurbreite" als die tatsächliche Höhe der Strokes 7, also des Teils der Scanlinie, auf der Laserfokuswirkbereiche 8 realisiert wurden bzw. werden, ist dann $2 sin \left(arccos\frac{1}{2}\right) = \sqrt{3} = 1 , 73$ also das 1,73-fache der Amplitude der Schwingung A: $A = \left‖\left(\begin{matrix}{A}_{x} \\ {A}_{y} \\ {A}_{z}\end{matrix}\right)\right‖ .$

Der Abstand zweier benachbarter "Schraffur"-Linien dT, 10 und damit zweier Strokes7 ist für den Fall, dass nur in einer Schwingungsrichtung, d.h.aufwärts 14 oder abwärts 15, geschnitten wird, über eine Schwingung konstant: $dT = \frac{v}{{f}_{L}} = \frac{1}{{f}_{L}} \left‖\left(\begin{matrix}{v}_{x} \\ {v}_{y} \\ {v}_{z}\end{matrix}\right)\right‖ .$

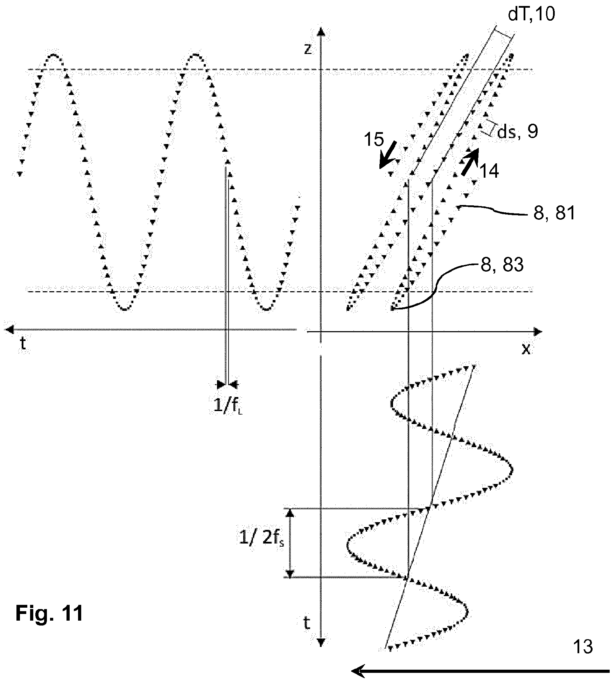

Die Fig. 11 zeigt die Überlagerung einer Sinus-Schwingung des schnellen z-Scanners mit einer synchronen Sinus-Schwingung eines schnellen lateralen Scanners in x-Richtung mit langsamer Veränderung des Schwingungsmittelpunktes in negative x-Richtung zur bidirektionalen Erzeugung von Strokes 7.

Im Gegensatz zum Beispiel der Fig. 10 werden hier die Strokes 7 auf einer geneigten Scanlinie 5 mit bidirektionalem Laserschnitt sowohl in der Aufwärtsbewegung 14 als auch in der Abwärtsbewegung 15 erzeugt, siehe Strokes 7 mit nach oben zeigenden Dreiecken für die Aufwärtsbewegung und Strokes 7 mit nach unten zeigenden Dreiecken für die Abwärtsbewegung, um die entsprechenden Laserfokuswirkbereiche 8 zu markieren.

Im bidirektionalen Schnittmodus variiert auch der Abstand zweier Strokes dT, 10 mit der jeweiligen Position auf der Scanlinie 5, ist in den Nulldurchgängen aber halb so groß wie beim unidirektionalen Schnittmodus der Fig. 10. Wird wie im unidirektionalen Fall bei $\sqrt{3} / 2$ der Amplitude beschnitten, womit der Abstand zweier Laserfokuswirkbereiche dS auf die Hälfte des Maximalwerts im Nulldurchgang fällt, variiert der Linienabstand und damit der Abstand dT, 10 zweier Strokes 7 abwechselnd von 1/3 und bis 5/3 des Wertes im Nulldurchgang. Der Abstand 2dT zur übernächsten, gleichgerichteten Scanlinie 5, und damit zum übernächsten Stroke 5, bleibt wie im unidirektionalen Modus konstant 6/3.

Sollen die Strokes 7 so eingebracht werden, dass nachfolgende, noch zu realisierende Strokes "über" den bisher realisierten Strokes 7 liegen, damit bereits realisierte Strokes 7 nicht den Fokuskegel 6 des Laserfokus 4 abschatten, muss bei positiver langsamer Bewegung in x-Richtung die Lateralschwingung, wie in der Fig. 10 vorliegend, gegenphasig zur z-Schwingung erfolgen, bei negativer langsamer Bewegung in x-Richtung, wie in der Fig. 11 vorliegend, muss die schnelle Lateralschwingung in x-Richtung gleichphasig zur schnellen z-Schwingung erfolgen.

Soll nun ein Capsulotomieschnitt ausgeführt werden, so besteht die Aufgabe, eine Schnittfläche 11 zu generieren, die die Haut der Linsenvorderseite eines Auges, die sogenannte "anterior Capsule" in einer wählbaren Lochgeometrie durchtrennt. Diese kann beispielsweise ellipsen- oder kreisförmig sein. Die Schnittfläche 11 soll sich dabei in einen minimalen Abstand sowohl über als auch unter den Kapselsack erstrecken, um ein sicheres Durchtrennen zu gewährleisten.

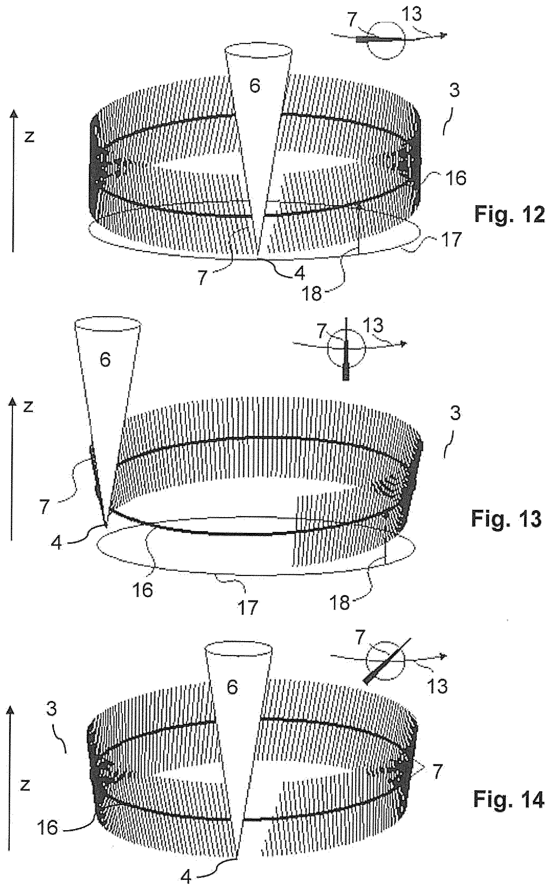

Zur Ausführung eines solchen Schnittes gibt es nun verschiedene mögliche Scanmuster für den Fokus 4 eines gepulsten Laserstrahls, der zum "Schneiden" verwendet wird. Eine erste Möglichkeit ist in der Fig. 12 dargestellt. Fig. 12 zeigt ein erstes Scanmuster zu Generierung eines Capsulotomieschnittes, in dem die langsame Bewegung lateral entlang der Lochgeometrie 17 und in z-Richtung der z-Höhe 18 des Kapselsacks folgt.

Eine schnelle oszillatorische Scanbewegung in z-Richtung ist synchronisiert zu schnellen oszillatorischen Scanbewegungen tangential zur lateralen langsamen Scanbewegung 13, also gleichgerichtet zur Lateralkomponente der langsamen Scanbewegung 13, zur Ausbildung der Strokes 7, also der auf einer Scanlinie 5 in gleicher Richtung realisierten Laserfokuswirkbereiche 8. Diese bewirken hierdurch Photodisruption eine Trennung des Augenmaterials 3 und tragen so zur Ausbildung einer Schnittfläche 11 bei. Es entsteht das Bild eines in "Zaunrichtung" geneigten "Lattenzauns".

Oben rechts in der Fig. 12 zeigt ein schematisches Bild in Draufsicht, dass die Strokes 7 in Richtung des Fortschrittes der langsamen Scanbewegung 13 ausgerichtet sind.

Das untere Ende eines solchen Strokes 7, dass dünn gezeichnet ist, eilt der langsamen Bewegung 13 voraus, das obere Ende, das dick gezeichnet ist, bleibt hinter der langsamen Bewegung 13 zurück, so dass der Fokuskegel 6 des Laserfokus 4 für den Laserfokuswirkbereiche 8 des nachfolgenden Stroke 7 nie durch bereits realisierte Strokes 7 abgeschattet wird. An der Stelle, wo das Ende der Schnittfläche 11 wieder den Anfang treffen soll, werden zu Beginn die Strokes 7 nach obenhin so begrenzt, dass ein V-förmiger Bereich frei bleibt zur Ausbildung der letzten Strokes 7, die dann die anfangs unvollständigen Strokes 7 ausfüllen.

Fig. 13 zeigt ein zweites Scanmuster zu Generierung eines Capsulotomieschnittes, in dem die langsame Bewegung lateral entlang der Lochgeometrie 17 und in z-Richtung der z-Höhe 18 des Kapselsacks folgt.

Eine schnelle oszillatorische Scanbewegung in z-Richtung ist synchronisiert zu einer lateralen schnellen oszillatorischen Scanbewegung normal zur lateralen langsamen Scanbewegung 13, also senkrecht zur Lateralkomponente der langsamen Scanbewegung 13, zur Ausbildung der Strokes 7, also der auf einer Scanlinie 5 in gleicher Richtung realisierten Laserfokuswirkbereiche 8. Diese bewirken auch hier durch Photodisruption eine Trennung des Augenmaterials 3 und tragen so zur Ausbildung einer Schnittfläche 11 bei. Es entsteht das Bild eines nach außen geneigten "Lattenzauns".

Oben rechts in der Fig. 13 zeigt ein schematisches Bild in Draufsicht, dass die Strokes 7 senkrecht zum Fortschritt der langsamen Scanbewegung 13 ausgerichtet sind. Sie lassen immer den Bereich für den Fokuskegel 6 des Laserfokus 4 für den nachfolgenden Stroke 7 frei, unabhängig davon, ob der Stroke 7 von der langsamen Scanbewegung 13 im inneren oder im äußeren Bereich nach oben zeigt, solange die in der Fig. 8c beschriebene Bedingung, dass der Neigungswinkel α der Strokes 7 größer ist als der Fokussierwinkel φ des Fokuskegels 6 des Laserfokus 4, realisiert ist.

Die Fig. 14 zeigt ein drittes Scanmuster zu Generierung eines Capsulotomieschnittes. Eine schnelle oszillatorische Scanbewegung in z-Richtung ist synchronisiert zu einer lateralen schnellen oszillatorischen Scanbewegung, die zur lateralen langsamen Scanbewegung 13 in einem Winkel zwischen tangentialer und normaler Richtung erfolgt. Diese führt zur Ausbildung der Strokes 7, die hier ebenfalls durch Photodisruption eine Trennung des Augenmaterials 3 bewirken und so zur Ausbildung einer Schnittfläche 11 beitragen. Dadurch entsteht das Bild schräg nach außen geneigter und gerade noch gehaltener "Mikadostäbchen".

Oben rechts in der Fig. 14 zeigt ein schematisches Bild in Draufsicht, dass die Strokes 7 in einem Winkel zwischen tangentialer und normaler Ausrichtung zum Fortschritt der langsamen Scanbewegung 13 ausgerichtet sind. Der der langsamen Scanbewegung 13 vorauseilende Teil der Strokes 7, der dünn gezeichnet ist, ist das tiefer liegende Ende des Strokes 7 und der dick gezeichnete Teil, der hinter der langsamen Scanbewegung 13 zurück bleibt, ist das obere Ende des Strokes 7. Dadurch wird der Fokuskegel 6 des Laserfokus 4 für den nachfolgenden Stroke 7 nicht durch bereits realisierten Stroke 7 abgeschattet, sofern die Bedingung, dass der Neigungswinkel α der Strokes 7 größer ist als der Fokussierwinkel φ des Fokuskegels 6 des Laserfokus 4, erfüllt ist.

Das Scanmuster der Fig. 12 ist das bevorzugte Scanmuster zu Generierung eines Capsulotomieschnittes. Hier bleibt die Lochgeometrie 17 im Wesentlichen auch dann erhalten, wenn der zu durchtrennende Kapselsack zum Zeitpunkt des Schnittes nicht mehr exakt an der geplanten Position liegt, was durch Ungenauigkeiten der Messung oder einer anschließende Bewegung der Augenmedien passieren kann, während bei den Scanmustern der Fig. 13 und 14 der Durchmesser des tatsächlich realisierten Capsulotomieschnitts von der tatsächlichen z-Position des Überlapps der Schnittfläche 11 mit dem Kapselsack, also eines transparenten Augenmaterials 3 abhängt.

In der Praxis erweist sich das Scanmuster der Fig. 12 auch als das mit der höchsten Schnitteffizienz, also der höchsten Schnittwirkung bei kleinsten Laserenergien und/oder größten Abständen 9 zweier Laserfokuswirkbereiche 8.

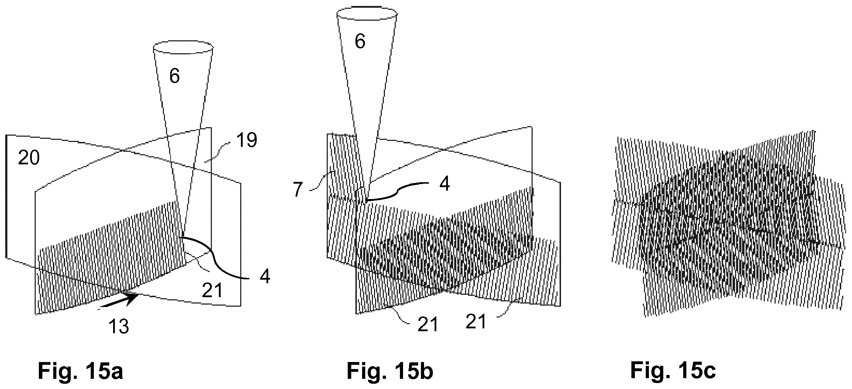

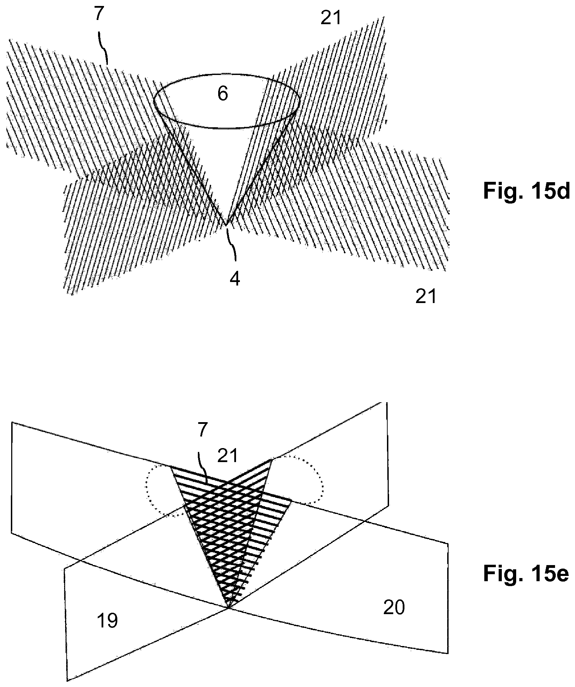

Die Fig. 15a bis 15c zeigen verschiedene Phasen eines Scanmusters zur Generierung eines Linsenfragmentationsschnittes, der wiederum durch Photodisruption mit Hilfe eines gepulsten Laserstrahl entsteht. Die Fig. 15d und 15e zeigen eine weitere Lösung für ein entsprechendes Scanmusters zur Vervollständigung der Schnittebenen an Kreuzungspunkten auf.

Der gepulste Laserstrahl wird hier wie auch für viele andere ophthalmologische Zwecke in der Regel mit einem Femtosekunden-Laser realisiert. Idealerweise wird beispielsweise für die Nutzung für augenchirurgische Zwecke hier wie auch in den Beispielen der Fig. 12, 13, 14, 16, 17 und 18 eine Anordnung zur Bearbeitung einer Fläche 11 in einem Bearbeitungsvolumen 300 eines transparenten Materials 3 genutzt, wie sie in der Fig. 3 beschrieben ist, also insbesondere eine Anordnung, die eine schnelle Scanbewegung in einem Teilbereich 600 des Bearbeitungsvolumens 300 unabhängig von einer langsamen Scanbewegung in beliebiger Richtung im gesamten Bearbeitungsvolumen 300 erlaubt, wobei der Teilbereich 600 der schnellen Scanbewegung durch die langsame Scanbewegung durch das Bearbeitungsvolumen 300 bewegt wird.

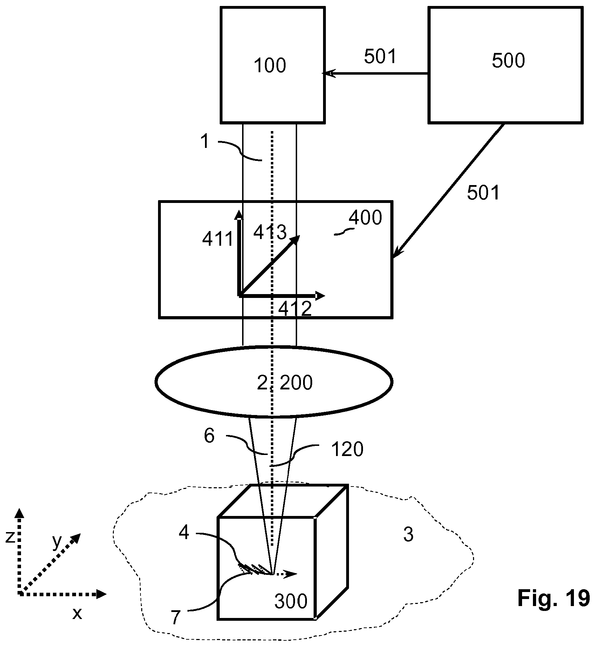

Es ist aber auch möglich, mit entsprechenden Anpassungen, etwa um das Zusammenwirken der schnellen Scanbewegung mit einer langsamen Scanbewegung zu simulieren und einem einzigen Scansystem, ggf. unter erheblichen Geschwindigkeitsverlust zu übertragen, andere Anordnungen zu nutzen, wie beispielsweise die Anordnung zur Bearbeitung einer Fläche 11 in einem Bearbeitungsvolumen 300 eines transparenten Materials 3, wie sie in der Fig. 19 beschrieben ist.

Zur Linsenfragmetation muss eine Schnittfläche 11 generiert werden, die eine Linse eines Auges entlang frei wählbarer Schnittebenen 19, 20 zerteilt. Die Schnittebenen 19, 20 sollen dabei Minimalabstände zu den Rändern der Linse einhalten, folgen daher oben und unten der Krümmung der begrenzenden Linsenflächen.

Für die Ausbildung einer ausgedehnten Schnittfläche 11, die das ganze Volumen der Linse durchtrennt, kann der Hub einer schnellen Scanbewegung, insbesondere der durch die Amplitude bestimmte Hub durch eine oszillatorische Bewegung einen schnellen z-Scanners, nicht ausreichen, um die Linse in kompletter Höhe zu zerteilen.

In diesem Fall muss die gesamte Schnittfläche aus mehreren Einzel-Schnittbändern 21 zusammengesetzt werden. Soll in einem Bearbeitungsvolumen 300 eine komplette Schnittebene 19, 20 erzeugt werden, deren Form also, anders als bei der Capsulotomie, wo nur die Durchdringung mit dem Kapselsack Relevanz hat, über die gesamte Ebene relevant ist, müssen die einzelnen Strokes 7 und damit die Scanlinien in dieser Schnittfläche 11 liegen. Die Strokes 7 müssen also zwangsläufig in Richtung des Fortschritts der langsamen Bewegung 13 oder entgegen der Richtung des Fortschritts der langsamen Bewegung 13 geneigt werden, also analog zum ersten Scanmuster der Capsulotomie.

Wiederum wird ein Teil der Laserpulse ausgeblendet, um eine Schädigung durch die Realisierung zweier Laserfokuswirkbereiche in nächster Nähe zueinander zu verhindern. Dabei werden die Laserpulse zusätzlich zu den Umkehrpunkten der schnellen oszillatorischen Scanbewegungen, die die schnellen Scanner ausführen, auch immer dann ausgeblendet, wenn die Scanlinie bei voller Amplitude der Oszillation den beabsichtigten Schnittbereich der Schnittebenen 19, 20 verlässt. Damit nicht ein einzelnes Schnittband 21 abschattend für ein folgendes Schnittband 21 wirkt, werden alle tiefliegenden Schnittbänder 21 zuerst erzeugt, bevor mit höherliegenden Bänden 21 fortgefahren wird , wie in der Fig. 15b gezeigt. Es erfolgt also ein etagenweiser Aufbau. Das komplettierte Schnittmuster der gesamten Schnittfläche 11 ist in Fig. 15c zu sehen. An den Durchdringungspunkten zweier Schnittbänder 21 kommt es ebenfalls zur Abschattung des nachfolgend realisierten Schnittbandes 21 durch das bereits realisierte Schnittband 21. Hier kann im Durchdringungspunkt ein kegelförmiger Bereich zunächst frei gelassen werden, der dann nach Abschluss der kompletten "Etage" alleine unter Verwendung der schnellen Scanner von unten nach oben gefüllt werden kann.

Die Fig. 15d und 15e verdeutlichen noch einmal das Problem, das bei Kreuzung zweier Schnittebenen, wie hier zweier gekreuzter Schnittebenen eines Linsenfragmetationsschnittes, auftritt und zeigen eine weitere Lösung für ein entsprechendes Scanmusters zur Vervollständigung der Schnittebenen an Kreuzungspunkten auf: Nachdem die sich kreuzenden Schnittbänder 21, wie in Fig. 15d gezeigt, mit einer trichterförmigen Aussparung am Kreuzungspunkt, die einem Fokuskegel 6 des Laserfokus 4 entspricht, erzeugt wurden, wird der Trichter durch ein Kreuzschnittband 21 in der trichterförmigen Aussparung ergänzt, das durch schnelle laterale oszillatorische Scanbewegungen und einer langsamen z-Scanbewegung erzeugt wird, wie aus der Fig. 15e deutlich wird.

In den Fig. 15a bis 15c sind übereinanderliegende Schnittbänder 21 in unterschiedliche Richtungen geneigt. Diese Neigungsrichtungen sind abhängig von der Fortschrittsrichtung der langsamen Scanbewegung 13. Die in den Fig. 15a bis 15c gewählten Fortschrittsrichtung der langsamen Scanbewegung 13 sind dabei nicht zwingend, sondern können in der Regel frei gewählt werden, allerdings führt das Vorliegen bereits realisierter Schnittbänder 21 ggf. zu einer bevorzugten Richtung der langsamen Scanbewegung 13 für ein noch zu realisierendes Schnittband 21. Übereinanderliegende Schnittbänder 21, die in gleicher Richtung geneigt sind, weil sie in gleicher langsamer Richtung 13 geschnitten werden, sind also ebenfalls im Rahmen der Erfindung.

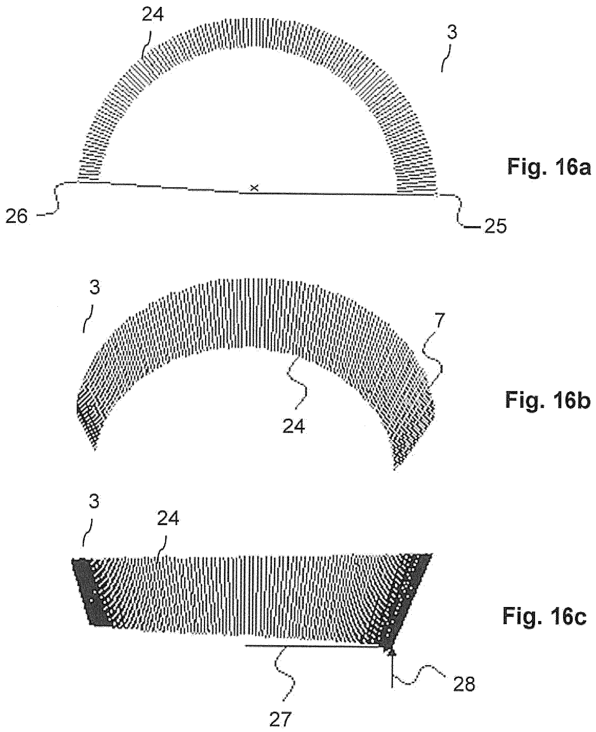

Ein Scanmuster zur Generierung einer Arcuate Incision, also eines bogenförmigen Schnitt-Profils, das beispielsweise zur Relaxation und Korrektur eines Astigmatismus eines Auges verwendet werden kann, ist in den Fig. 16a bis 16c in verschiedenen Ansichten dargestellt: Die Fig. 16a zeigt eine Draufsicht, die Fig. 16b eine schräge Projektion und die Fig. 16c eine Seitenansicht eines Scanmusters bzw. Schnittmusters, das für eine Arcuate Incision geeignet ist.

Für eine Arcuate Incision muss eine Schnittfläche in Form eines schrägen, gebogenen Schnittbandes 24 generiert werden. Dies wird analog der Scanmuster der Fig. 13 und 14 für die Capsulotomie gelöst, wobei die Schräglage, also die Neigung jedes Strokes 7 kontinuierlich verändert werden kann, die Anfangs- und Endpunkte, also beispielsweise die Höhe in z-Richtung, bei der das entsprechende Stroke 7 beginnt bzw. endet, kontinuierlich variiert werden können, und Startwinkel 25 sowie Endwinkel 26 des gebogenen Schnittbandes 24 frei wählbar sind.

Die Strokes 7 verlaufen, wie in der Draufsicht der Fig. 16a gezeigt, radial von einer Achse ausgehend.

Das Schnittband 24 beginnt bei einem Startwinkel 25 und endet bei einem Endwinkel 26. Der Start- und Endpunkt eines jeden Strokes 7 an seiner jeweiligen Winkelposition, ist beispielsweise gegeben durch Abstand von der Achse 27 und einer z-Höhe 28 und kann sich beim Durchlaufen des Winkelbereichs vom Startwinkel 25 zum Endwinkel 26 kontinuierlich ändern. Damit kann die Neigung, die Länge und die Position der Strokes 7 der Geometrie des zu schneidenden Augengewebes 3angepasst werden. Da bei dieser Anwendung das Schnittband bzw. die Schnittfläche 24, anders als bei der Capsulotomie, im Volumen des Augengewebes 3 erzeugt werden soll, ist die freie Wahlmöglichkeit der Form der Schnittfläche 24 hier von großer Bedeutung.

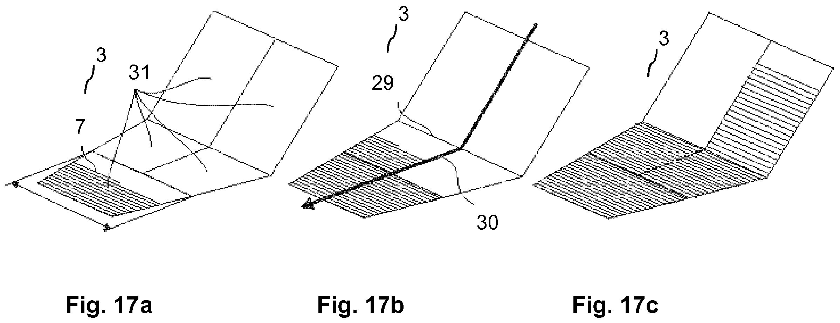

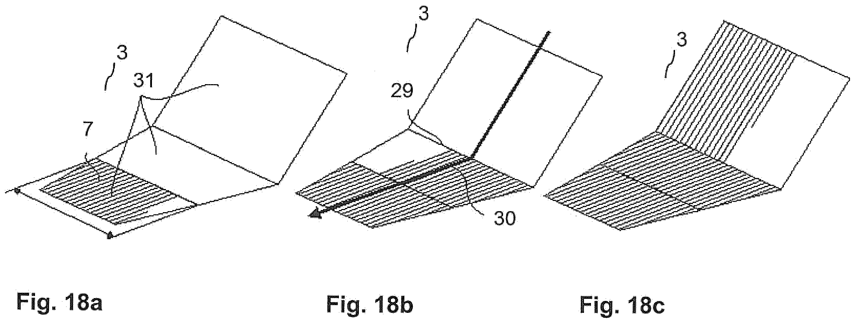

Ein erstes und ein zweites Scanmuster zu Generierung einer Access Incision, also eines Zugangsschnitts im Rahmen eines ophthalmologischen Therapieverfahrens wird schlussendlich in den Fig. 17a bis 17c sowie Fig. 18a bis 18c in verschiedenen Phasen dargestellt.

Ein solcher Zugangsschnitt bzw. "Zugangstunnel" soll mit einer gewünschten Breite, Neigung und mit entsprechenden "Knickkanten" 29 zur Ausbildung einer selbstdichtenden Schnittgeometrie durch die Cornea des Auges 3 generiert werden.

Auch wenn eine Access Incision mit schrägen Strokes 7 ausgeführt werden kann, erfordert sie nicht zwingend einen z-Anteil der schnellen oszillatorischen Scanbewegung, da der Scan senkrecht zur Zugangsrichtung 30 wie in der Fig. 17a, oder parallel zur Zugangsrichtung 30 wie in der Fig. 18a lateral in einer x-y Ebene ausgeführt werden kann.

Mitunter ist die Amplitude der lateralen oszillatorischen Scanbewegung kleiner als die erforderliche Breite des Tunnels. Die Schnittfläche muss dann in Teilbereiche 31 zerlegt werden, die sequentiell ausgeführt werden.

Insbesondere der in der Fig. 17a begonnene Teilbereich 31 erreicht am Ende die maximale Breite der ozillatorischen Scanbewegung. In der Phase der Fig. 17b wird daher der Tunnel in zwei nebeneinanderliegende Bereiche 31 aufgeteilt und hintereinander abgearbeitet. Eine weitere Unterteilung, z.B. an Knickkanten 29 kann vorteilhaft sein.

Wie in den Fig. 17c und 18c gezeigt, wird erst nach Fertigstellung des ebenen Bereichs, der in einer x-y-Ebene bearbeitet wurde, mit der nächsten Ebene begonnen. Das langsame Scansystem kann dabei während der Ausführung der Strokes 7 an fester Position verweilen und nur im Anschluss an die Fertigstellung eines Teilbereiches zur nächsten Position fahren: Der langsame Lateralscanner muss für ein Voranschreiten während der Bearbeitung einer Schnittfläche 31 dafür nicht benutzt werden: Dies kann auch durch eine langsame Nulllagenverschiebung der Oszillation der schnellen Scanner erfolgen.

Bei Erzeugung der geneigten Ebene ist auch wiederum die Beachtung der Größe des Neigungswinkels im Vergleich zum Fokussierwinkel nötig, um Abschattungseffekte zu vermeiden. Ggf muss auch hier schräg aufgefüllt werden, wie bereits für Fig. 14 und 15b vorgeschlagen und in Fig. 9 dargestellt.

Durch die langsame Bewegung des Oszillationszentrums in Zugangsrichtung werden die einzelnen Strokes 7 nebeneinander realisiert. Durch Ausblenden der Fokuswirkbereiche 8 der Laserpulse dann, wenn die Oszillationsbewegung die Ränder der Teilbereiche überschreitet, wird die Formtreue der Schnittfläche 11, 31 mit beliebig wählbarem Breitenverlauf über die Zugangsrichtung 30 gewährleistet.

Die Fig. 19 zeigt eine zweites Beispiel einer Anordnung zur Bearbeitung einer Fläche in einem Bearbeitungsvolumen eines transparenten Materials mittels einer fokussierten Strahlung.

Die Anordnung enthält eine Einrichtung zur Erzeugung einer Strahlung 100, die einen Femtosekunden-Laser mit einer Wellenlänge im Bereich von 1020 - 1060nm umfasst. Die Pulsdauer dieses Femtosekunden-Lasers beträgt 500-600fs, die Pulsenergie ca. 10µJ.

Des Weiteren enthält die Anordnung eine Optik 2, 200 zur Fokussierung der Strahlung in einem Fokus 4, mit einer numerischen Apertur von 0,2, die eine Bildfeldgröße von ca. 6mm aufweist. Mit dieser Anordnung ist ein Bearbeitungsvolumen 600 von 6mm x 6mm x 6mm erreichbar.

Die hier gezeigte Anordnung enthält ein Einrichtung zur Veränderung der Lage 400 des Fokus 4 mit einem Scansystem, das in x-, y und z-Richtung Scanbewegungen ausführen kann und durch die Zusammensetzung dieser Scanbewegungen eine Scanbewegung in beliebiger Richtung vollführen kann. Die Einrichtung zur Veränderung der Lage 400 enthält hierzu drei Scanner 411, 412, 413, die ggf. die Optik entsprechend verschieben können. Da sie jedoch kein zweites, vom ersten Scansystem unabhängig einstellbares schnelles Scansystem enthält, sind mit einer solchen Anordnung die hier vorgestellten Scanmuster nur unter Zeitverlust realisierbar.

Des Weiteren enthält die Anordnung ein einteiliges zentrales Steuersystem 500, das über Kommunikationswege 501 mit der Einrichtung zur Erzeugung einer Strahlung 100, also dem Femtosekunden-Lasersystem, und mit der Einrichtung zur Veränderung der Lage 400 des Fokus 4 verbunden ist und eingerichtet ist, sowohl das Femtosekunden-Lasersystem als auch alle Scanner 411, 412, 413 der Einrichtung zur Veränderung der Lage 400 des Fokus 4 zu steuern.

Trotz eines Fehlens eines zusätzlichen schnellen Scansystems ist es mit einem solchen Scansystem möglich, vorteilhafte Scanmuster derart zu realisieren, dass es nicht oder kaum zu Abschattungswirkungen durch bereits realisierte Teile des Scanmusters für noch zu realisierende Teile des Scanmusters kommt, wenn entsprechende Scanmuster in die Steuereinrichtung 500 dieser Anordnung kodiert sind. Dabei ist ein Arbeiten, das - wie hier realisiert - in jedem Moment sehr lokal beschränkt ist, beispielsweise in der Augenchirurgie günstig, um die Auswirkungen einer möglichen Bewegung des Auges während des Eingriffs in das Augengewebe 3 gering zu halten.

Der Ansteuerwert für jede Scanrichtung setzt sich dabei aus langsamen, weitreichenden Grundkomponenten in den drei Raumrichtungen mit in diesen Grenzen beliebigem Zeitverlauf und kurzreichweitigen, schnell repetierenden Komponenten, die synchronen Richtungswechselbewegungen in den drei Raumrichtungen entsprechen und deren Zeitverlauf sich bei jeder Repetition nur wenig verändert, zusammen.

Die vorstehend genannten und in verschiedenen Ausführungsbeispielen erläuterten Merkmale der Erfindung sind dabei nicht nur in den beispielhaft angegebenen Kombinationen, sondern auch in anderen Kombinationen oder allein einsetzbar, ohne den Rahmen der vorliegenden Erfindung zu verlassen.

## Patentansprüche

1. Ophthalmologische Therapieanordnung zur Bearbeitung einer Fläche (11) in einem Bearbeitungsvolumen (300) eines transparenten Materials (3) eines Auges mittels einer fokussierten Strahlung (1), umfassend
- eine Einrichtung zur Erzeugung (100) einer Strahlung (1),
- eine Optik zur Fokussierung (2, 200, 202) der Strahlung (1) in einem Fokus (4) im Bearbeitungsvolumen (300), wobei der Fokus (4) der fokussierten Strahlung einen Fokussierwinkel (φ) und die fokussierte Strahlung eine Strahlachse (12) aufweist,
- eine Einrichtung zur Veränderung der Lage (400) des Fokus (4) im Bearbeitungsvolumen (300), das mit drei Raumrichtungen x, y und z beschreibbar ist, wobei die z-Richtung parallel zu einer Grundstellungs-Strahlachse (120) der fokussierten Strahlung (12) verläuft,
- eine Steuereinrichtung (500) zur Steuerung der ophthalmologischen Therapieanordnung,
- **dadurch gekennzeichnet, dass** die Einrichtung zur Veränderung der Lage (400) des Fokus (4) eingerichtet ist, in jeder beliebigen, durch die drei Raumrichtungen bestimmten Richtung eine langsame Scanbewegung im Bearbeitungsvolumen (300) des transparenten Materials (3) und in jeder beliebigen, durch die drei Raumrichtungen bestimmten Richtung eine von der langsamen Scanbewegung unabhängige schnelle Scanbewegung in einem Teilbereich (600) des Bearbeitungsvolumens (300) zu vollziehen,
- wobei der Teilbereich (600) der schnellen Scanbewegung durch die langsame Scanbewegung im gesamten Bearbeitungsvolumen (300) bewegbar ist.

2. Ophthalmologische Therapieanordnung nach Anspruch 1, **gekennzeichnet durch** eine Einrichtung zur Erzeugung (100) einer Strahlung (1), die einen Laser umfasst.

3. Ophthalmologische Therapieanordnung nach Anspruch 2, wobei der Laser ein gepulster Laser, insbesondere ein Kurzpulslaser, bevorzugt ein Femtosekunden-Laser ist.

4. Ophthalmologische Therapieanordnung nach einem der Ansprüche 1 bis 3, wobei in die Steuereinrichtung (500) ein Scanmuster kodiert ist, das eine Abfolge von Fokuswirkbereichen (8, 81, 82) der fokussierten Strahlung (1) entlang einer Scanlinie (5) im Bearbeitungsvolumen (300) aufweist, derart dass bereits realisierte Fokuswirkbereiche (8, 81) stets außerhalb eines Fokuskegels (6), der durch den Fokus (4) der fokussierten Strahlung (1) und den Fokussierwinkel (φ) gebildet ist, für noch zu realisierende Fokuswirkbereiche (8, 82) angeordnet sind.

5. Ophthalmologische Therapieanordnung nach einem der Ansprüche 1 bis 4, **gekennzeichnet durch** eine Einrichtung zur Veränderung der Lage (400) des Fokus (4), die einen schnellen z-Scanner (401) und mindestens einen schnellen Lateralscanner (402, 403) zur Realisierung der schnellen Scanbewegung im Teilbereich (600) des Bearbeitungsvolumens (300) zusätzlich zu einem langsamen z-Scanner (411) und mindestens einem langsamen Lateralscanner (412, 413) zur Realisierung der langsamen Scanbewegung im Bearbeitungsvolumen (300) enthält.

6. Ophthalmologische Therapieanordnung nach Anspruch 5, **gekennzeichnet durch** einen schnellen Lateralscanner (402, 403), der durch einen schnellen x-Scanner (402) ausgebildet ist und neben einem weiteren schnellen Lateralscanner vorliegt, der durch einen schnellen y-Scanner (403) ausgebildet ist, oder durch einen schnellen Lateralscanner (402, 403), der durch einen schnellen R-Scanner ausgebildet ist, dessen Scanbewegung in einer x-y-Ebene senkrecht zur Grundstellungs-Strahlachse (120) durch Drehung um eine zur Grundstellungs-Strahlachse (120) parallele Drehachse ausrichtbar ist.

7. Ophthalmologische Therapieanordnung nach Anspruch 5 oder 6, **gekennzeichnet durch** einen schnellen z-Scanner, der ein in z-Richtung oszillierendes Linsenelement umfasst, einen schnellen Lateralscanner, der ein um zwei Achsen bewegliches x-y-Spiegelelement oder zwei einzelne jeweils um eine Achse bewegliche Spiegelelemente, deren Achsen bevorzugt senkrecht zueinander stehen, umfasst, einen langsamen z-Scanner, der ein in z-Richtung wahlfrei bewegliches Linsenelement umfasst und einen langsamen Lateralscanner, der eine wahlfrei in der lateralen Ebene verschiebbare Fokussieroptik umfasst.

8. Ophthalmologische Therapieanordnung nach einem der Ansprüche 5 bis 7, eingerichtet zur Ausführung einer schnellen Scanbewegung durch synchrone Richtungswechsel mindestens zweier schneller Scanner (401, 402, 403), insbesondere durch synchrone oszillatorische Bewegungen mindestens zweier schneller Scanner (401, 402, 403).

9. Ophthalmologische Therapieanordnung nach Anspruch 8, **dadurch gekennzeichnet, dass** einer der mindestens zwei schnellen Scanner (401, 402, 403) ein resonanter Scanner mit einer freien Oszillation ist, und alle weiteren der mindestens zwei schnellen Scanner (401, 402, 403) auf den resonanten Scanner synchronisiert sind.

10. Ophthalmologische Therapieanordnung nach Anspruch 8 oder 9, **gekennzeichnet durch** synchrone oszillatorische Bewegungen des schnellen z-Scanners (401) und des mindestens einen schnellen Lateralscanner (402, 403).

11. Ophthalmologische Therapieanordnung nach Anspruch 10, wobei die langsame Scanbewegung eine Lateralkomponente (13) in x-Richtung und/oder in y-Richtung aufweist und die oszillatorischen Bewegungen des schnellen z-Scanners (401) und des mindestens einen schnellen Lateralscanners (402, 403) so synchronisiert sind, dass
- bei einer positiven Lateralkomponente (13) der langsamen Scanbewegung in x- und/oder y-Richtung die oszillatorischen Bewegungen des schnellen Lateralscanners, insbesondere des schnellen x-Scanners und/oder des schnellen y-Scanners, gegenphasig zur oszillatorischen Bewegung des schnellen z-Scanners sind, und dass
- bei einer negativen Lateralkomponente (13) der langsamen Scanbewegung in x- und/oder y-Richtung die oszillatorischen Bewegungen des schnellen Lateralscanners, insbesondere des schnellen x-Scanners und/oder des schnellen y-Scanners, gleichphasig zur oszillatorischen Bewegung des schnellen z-Scanners sind.

12. Ophthalmologische Therapieanordnung nach einem der Ansprüche 4 bis 11, **gekennzeichnet durch** eine Steuereinrichtung (500), in die ein Scanmuster kodiert ist, das zueinander benachbarte Strokes (7) mit Neigungswinkeln (α) zur Strahlachse (12) aufweist, wobei ein Stroke (7) einen gleichgerichteten Teil einer Scanlinie (5) umfasst und durch eine Aneinanderreihung von Fokuswirkbereichen (8) der fokussierten Strahlung (1) realisiert ist, und wobei die Neigungswinkel (α) der Strokes (7) zur Strahlachse (12) stets größer oder gleich dem Fokussierwinkel (φ) der fokussierten Strahlung (1) sind.

13. Ophthalmologische Therapieanordnung nach Anspruch 12, **gekennzeichnet durch** eine Kodierung der Steuereinrichtung (500) derart, dass die Ausbildung der Strokes (7) durch eine Aneinanderreihung von Fokuswirkbereichen (8) der fokussierten Strahlung (1) stets in einer Aufwärtsbewegung (14) oder stets in einer Abwärtsbewegung (15) oder abwechselnd in einer Aufwärtsbewegung (13) und in einer Abwärtsbewegung (14) entlang der Scanlinie (5) realisiert ist.

14. Ophthalmologische Therapieanordnung nach einem der Ansprüche 1 bis 13, wobei die Einrichtung zur Erzeugung einer Strahlung einen gepulsten Laser mit einer Laserpulsrepetitionsrate (f_{L}) umfasst und ein Abstand (9) eines Fokuswirkbereichs (8) von einem vorangehenden Fokuswirkbereich (8) durch die Laserpulsrepetitionsrate (f_{L}) und eine Gesamtscangeschwindigkeit, die sich aus den Scangeschwindigkeiten der langsamen und schnellen Scanbewegungen zusammensetzt, bestimmt ist, **gekennzeichnet durch** eine Steuereinrichtung (500), die eingerichtet ist, einen Laserpuls auszublenden, wenn dessen Fokuswirkbereich (8) einen Mindestabstand (9) zum vorangehenden Fokuswirkbereich (8) unterschreitet.

15. Ophthalmologische Therapieanordnung zur Bearbeitung einer Fläche (11) in einem Bearbeitungsvolumen (300) eines transparenten Materials (3) eines Auges mittels einer fokussierten Strahlung (1), umfassend
- eine Einrichtung zur Erzeugung (100) einer Strahlung (1),
- eine Optik (2, 200, 201, 202) zur Fokussierung der Strahlung (1) in einem Fokus (4) im Bearbeitungsvolumen (300), wobei der Fokus (4) der fokussierten Strahlung einen Fokussierwinkel (φ) und die fokussierte Strahlung eine Strahlachse (12) aufweist,
- eine Einrichtung zur Veränderung der Lage (400) des Fokus (4) mittels einer Scanbewegung in beliebiger änderbarer Richtung im Bearbeitungsvolumen (300), das mit drei Raumrichtungen x, y und z beschreibbar ist, wobei die z-Richtung parallel zu einer Grundstellungs-Strahlachse (120) verläuft,
- **gekennzeichnet durch** eine Steuereinrichtung (500) zur Steuerung der Anordnung, in die ein Scanmuster kodiert ist, das durch die Scanbewegung entlang einer Scanlinie (5) realisierbar ist,
wobei die Scanbewegung mindestens eine laterale Grundkomponente (13) in x- und/oder y-Richtung aufweist, die überlagert ist von Komponenten synchroner Richtungswechselbewegungen in z-Richtung und in x-Richtung und/oder y-Richtung, wobei die synchronen Richtungswechselbewegungen so aufeinander synchronisiert sind, dass bei einer positiven lateralen Grundkomponente (13) der Scanbewegung in x- und/oder y-Richtung die Richtungswechselbewegungen in x- und/oder y-Richtung gegenläufig zu den Richtungswechselbewegungen in z-Richtung sind, und dass bei einer negativen lateralen Grundkomponente (13) der Scanbewegung in x- und/oder y-Richtung die Richtungswechselbewegungen in x- und/oder y-Richtung gleichläufig zu den Richtungswechselbewegungen in z-Richtung sind.

16. Ophthalmologische Therapieanordnung nach Anspruch 15, **gekennzeichnet durch** ein Scanmuster, das zueinander benachbarte Strokes (7) mit Neigungswinkeln (α) zur Strahlachse (12) aufweist, wobei ein Stroke (7) einen gleichgerichteten Teil der Scanlinie (5) umfasst und durch eine Aneinanderreihung von Fokuswirkbereichen (8) der fokussierten Strahlung (1) realisiert ist, wobei die Neigungswinkel (α) des Strokes größer oder gleich dem Fokussierwinkel (φ) der fokussierten Strahlung (1) sind.

17. Steuerprogrammprodukt, eingerichtet zur Kodierung einer Steuereinrichtung (500) einer ophthalmologischen Therapieanordnung zur Bearbeitung einer Fläche (11) in einem Bearbeitungsvolumen (300) eines transparenten Materials (3) eines Auges mittels einer fokussierten Strahlung (1) nach einem der Ansprüche 1 bis 16, die
- eine Einrichtung zur Erzeugung (100) einer Strahlung (1),
- eine Optik (2, 200, 201, 202) zur Fokussierung der Strahlung (1) in einem Fokus (4) im Bearbeitungsvolumen (300), das mit drei Raumrichtungen x, y und z beschreibbar ist, enthält, wobei der Fokus (4) der fokussierten Strahlung einen Fokussierwinkel (φ) und die fokussierte Strahlung eine Strahlachse (12), und
- eine Einrichtung zur Veränderung der Lage (400) des Fokus (4) im Bearbeitungsvolumen (300), das mit drei Raumrichtungen x, y und z beschreibbar ist, wobei die z-Richtung parallel zu einer Grundstellungs-Strahlachse (120) der fokussierten Strahlung (12) verläuft, aufweist,
wobei die Steuereinrichtung so kodiert wird,
- dass die Lage des Fokus (4) durch eine langsame Scanbewegung in jeder beliebigen, durch die drei Raumrichtungen bestimmten Richtung im Bearbeitungsvolumen (300) des transparenten Materials (3) und eine von der langsamen Scanbewegung unabhängige schnelle Scanbewegung in einem Teilbereich (600) des Bearbeitungsvolumens (300) in jeder beliebigen, durch die drei Raumrichtungen bestimmten Richtung verändert wird, und wobei der Teilbereich (600) der schnellen Scanbewegung durch die langsame Scanbewegung im gesamten Bearbeitungsvolumen (300) bewegt wird, oder
- dass die Lage des Fokus (4) der fokussierten Strahlung durch eine Scanbewegung im Bearbeitungsvolumen (300) des transparenten Materials (3) in jeder beliebigen, durch die drei Raumrichtungen x, y und z bestimmten Richtung verändert wird, und ein Scanmuster durch die Scanbewegung im transparenten Material (3) erzeugt wird, derart, dass eine Scanbewegung mit einer lateralen Grundkomponente (13) in x-Richtung und/oder in y-Richtung überlagert wird von Komponenten synchroner Richtungswechselbewegungen in z-Richtung und in x-Richtung und/oder y-Richtung, wobei die Richtungswechselbewegungen so aufeinander synchronisiert werden, dass bei einer positiven lateralen Grundkomponente (13) der Scanbewegung in x- und/oder y-Richtung die Richtungswechselbewegungen in x- und/oder y-Richtung gegenläufig zu den Richtungswechselbewegungen in z-Richtung erfolgen, und dass bei einer negativen lateralen Grundkomponente (13) der Scanbewegung in x- und/oder y-Richtung die Richtungswechselbewegungen in x- und/oder y-Richtung gleichläufig zu den Richtungswechselbewegungen in z-Richtung erfolgen.

18. Planungseinheit, die in einer Steuereinrichtung (500) einer ophthalmologischen Therapieanordnung zur Bearbeitung einer Fläche (11) in einem Bearbeitungsvolumen (300) eines transparenten Materials (3) eines Auges mittels einer fokussierten Strahlung (1) enthalten ist oder die als selbständige Einheit der Steuereinrichtung (500) eingerichtet ist, mit anderen Einheiten der Steuereinrichtung (500) über Kommunikationswege (501) in Verbindung zu stehen,
- wobei die ophthalmologische Therapieanordnung des Weiteren
- eine Einrichtung zur Erzeugung (100) einer Strahlung (1),
- eine Optik (2, 200, 201, 202) zur Fokussierung der Strahlung (1) in einem Fokus (4) im Bearbeitungsvolumen (300), das mit drei Raumrichtungen x, y und z beschreibbar ist, enthält, wobei der Fokus (4) der fokussierten Strahlung einen Fokussierwinkel (φ) und die fokussierte Strahlung eine Strahlachse (12) und
- eine Einrichtung zur Veränderung der Lage (400) des Fokus (4) im Bearbeitungsvolumen (300), das mit drei Raumrichtungen x, y und z beschreibbar ist,
wobei die z-Richtung parallel zu einer Grundstellungs-Strahlachse (120) der fokussierten Strahlung (12) verläuft, aufweist,
wobei die Planungseinheit eingerichtet ist, alle Schritte einer Therapieführung zur Durchführung eines Verfahrens zur Bearbeitung einer Fläche (11) in einem Bearbeitungsvolumen (300) eines transparenten Materials (3) eines Auges durch eine fokussierte Strahlung (1) so zu vorzusehen,
- dass die Lage des Fokus (4) durch eine langsame Scanbewegung in jeder beliebigen, durch die drei Raumrichtungen bestimmten Richtung im Bearbeitungsvolumen (300) des transparenten Materials (3) und eine von der langsamen Scanbewegung unabhängige schnelle Scanbewegung in einem Teilbereich (600) des Bearbeitungsvolumens (300) in jeder beliebigen, durch die drei Raumrichtungen bestimmten Richtung verändert wird, und wobei der Teilbereich (600) der schnellen Scanbewegung durch die langsame Scanbewegung im gesamten Bearbeitungsvolumen (300) bewegt wird, oder
- dass die Lage des Fokus (4) der fokussierten Strahlung durch eine Scanbewegung im Bearbeitungsvolumen (300) des transparenten Materials (3) in jeder beliebigen, durch die drei Raumrichtungen x, y und z bestimmten Richtung verändert wird, und ein Scanmuster durch die Scanbewegung im transparenten Material (3) erzeugt wird, derart, dass eine Scanbewegung mit einer lateralen Grundkomponente (13) in x-Richtung und/oder in y-Richtung überlagert wird von Komponenten synchroner Richtungswechselbewegungen in z-Richtung und in x-Richtung und/oder y-Richtung, wobei die Richtungswechselbewegungen so aufeinander synchronisiert werden, dass bei einer positiven lateralen Grundkomponente (13) der Scanbewegung in x- und/oder y-Richtung die Richtungswechselbewegungen in x- und/oder y-Richtung gegenläufig zu den Richtungswechselbewegungen in z-Richtung erfolgen, und dass bei einer negativen lateralen Grundkomponente (13) der Scanbewegung in x- und/oder y-Richtung die Richtungswechselbewegungen in x- und/oder y-Richtung gleichläufig zu den Richtungswechselbewegungen in z-Richtung erfolgen.

19. Planungseinheit nach Anspruch 18, die eine Auswahltabelle von Scanmustern und/oder einen Algorithmus zur Erstellung eines Scanmusters enthält.

## Claims

1. Ophthalmological therapy assembly for processing a surface (11) in a processing volume (300) of a transparent material (3) of an eye by means of focused radiation (1), comprising
- a device for generating (100) radiation (1),
- an optical unit for focusing (2, 200, 202) the radiation (1) in a focus (4) in the processing volume (300), wherein the focus (4) of the focused radiation has a focusing angle (φ) and the focused radiation has a beam axis (12),
- a device for changing the position (400) of the focus (4) in the processing volume (300) that is describable with three spatial directions x, y and z, wherein the z-direction extends parallel to a basic-position beam axis (120) of the focused radiation (12),
- a control device (500) for controlling the ophthalmological therapy assembly,
- **characterized in that** the device for changing the position (400) of the focus (4) is configured to perform a slow scanning movement in the processing volume (300) of the transparent material (3) in every desired direction determined by the three spatial directions, and to perform a fast scanning movement, independent of the slow scanning movement, in a partial region (600) of the processing volume (300) in every desired direction determined by the three spatial directions,
- wherein the partial region (600) of the fast scanning movement is movable owing to the slow scanning movement in the entire processing volume (300).

2. Ophthalmological therapy assembly according to Claim 1, **characterized by** a device for generating (100) radiation (1), comprising a laser.

3. Ophthalmological therapy assembly according to Claim 2, wherein the laser is a pulsed laser, in particular a short pulse laser, preferably a femtosecond laser.

4. Ophthalmological therapy assembly according to one of Claims 1 to 3, wherein a scanning pattern is coded into the control device (500), which scanning pattern has a sequence of focus effective regions (8, 81, 82) of the focused radiation (1) along a scan line (5) in the processing volume (300) so that already realized focus effective regions (8, 81) are always arranged outside a focus cone (6), which is formed by the focus (4) of the focused radiation (1) and the focusing angle (φ), for focus effective regions (8, 82) that are still to be realized.

5. Ophthalmological therapy assembly according to one of Claims 1 to 4, **characterized by** a device for changing the position (400) of the focus (4), containing a fast z-scanner (401) and at least one fast lateral scanner (402, 403) for realizing the fast scanning movement in the partial region (600) of the processing volume (300) in addition to a slow z-scanner (411) and at least one slow lateral scanner (412, 413) for realizing the slow scanning movement in the processing volume (300).

6. Ophthalmological therapy assembly according to Claim 5, **characterized by** a fast lateral scanner (402, 403), which is formed by a fast x-scanner (402) and is present in addition to a further fast lateral scanner, which is formed by a fast y-scanner (403), or by a fast lateral scanner (402, 403), which is formed by a fast R-scanner whose scanning movement in an x-y plane perpendicular to the basic-position beam axis (120) is orientable by a rotation about an axis of rotation parallel to the basic-position beam axis (120).

7. Ophthalmological therapy assembly according to Claim 5 or 6, **characterized by** a fast z-scanner comprising a lens element oscillating in the z-direction, a fast lateral scanner comprising an x-y mirror element that is movable about two axes, or two individual mirror elements that are each movable about one axis and whose axes are preferably perpendicular to one another, a slow z-scanner comprising a lens element optionally movable in the z-direction, and a slow lateral scanner comprising a focusing optical unit displaceable optionally in the lateral plane.

8. Ophthalmological therapy assembly according to one of Claims 5 to 7, configured for performing a fast scanning movement by way of synchronous direction changes of at least two fast scanners (401, 402, 403), in particular by way of synchronous oscillatory movements of at least two fast scanners (401, 402, 403).

9. Ophthalmological therapy assembly according to Claim 8, **characterized in that** one of the at least two fast scanners (401, 402, 403) is a resonant scanner with a free oscillation, and all further scanners of the at least two fast scanners (401, 402, 403) are synchronized with respect to the resonant scanner.

10. Ophthalmological therapy assembly according to Claim 8 or 9, **characterized by** synchronous oscillatory movements of the fast z-scanner (401) and of the at least one fast lateral scanner (402, 403).

11. Ophthalmological therapy assembly according to Claim 10, wherein the slow scanning movement has a lateral component (13) in the x-direction and/or in the y-direction, and the oscillatory movements of the fast z-scanner (401) and of the at least one fast lateral scanner (402, 403) are synchronized such that,
- in a positive lateral component (13) of the slow scanning movement in the x-direction and/or y-direction, the oscillatory movements of the fast lateral scanner, in particular of the fast x-scanner and/or of the fast y-scanner, are in phase opposition with respect to the oscillatory movement of the fast z-scanner, and that,
- in a negative lateral component (13) of the slow scanning movement in the x-direction and/or y-direction, the oscillatory movements of the fast lateral scanner, in particular of the fast x-scanner and/or of the fast y-scanner, are in phase with respect to the oscillatory movement of the fast z-scanner.

12. Ophthalmological therapy assembly according to one of Claims 4 to 11, **characterized by** a control device (500) into which a scanning pattern is coded which has mutually adjacent strokes (7) having inclination angles (α) with respect to the beam axis (12), wherein a stroke (7) comprises an aligned part of a scan line (5) and is realized by a connected series of focus effective regions (8) of the focused radiation (1), and wherein the inclination angles (α) of the strokes (7) with respect to the beam axis (12) are always greater than or equal to the focusing angle (φ) of the focused radiation (1).

13. Ophthalmological therapy assembly according to Claim 12, **characterized by** a coding of the control device (500) such that the formation of the strokes (7) by way of a connected series of focus effective regions (8) of the focused radiation (1) is always realized in an up-movement (14) or is always realized in a down-movement (15) or is realized in an up-movement (13) and in a down-movement (14) in alternation along the scan line (5).

14. Ophthalmological therapy assembly according to one of Claims 1 to 13, wherein the device for generating radiation comprises a pulsed laser with a laser pulse repetition rate (f_{L}) and a distance (9) of a focus effective region (8) from a preceding focus effective region (8) is determined by the laser pulse repetition rate (f_{L}) and a total scanning speed, which is composed of the scanning speeds of the slow and fast scanning movements, **characterized by** a control device (500), which is configured to block out a laser pulse if its focus effective region (8) falls below a minimum distance (9) from the preceding focus effective region (8).

15. Ophthalmological therapy assembly for processing a surface (11) in a processing volume (300) of a transparent material (3) of an eye by means of focused radiation (1), comprising
- a device for generating (100) radiation (1),
- an optical unit (2, 200, 201, 202) for focusing the radiation (1) in a focus (4) in the processing volume (300), wherein the focus (4) of the focused radiation has a focusing angle (φ) and the focused radiation has a beam axis (12),
- a device for changing the position (400) of the focus (4) by means of a scanning movement in an arbitrarily changeable direction in the processing volume (300) that is describable with three spatial directions x, y and z, wherein the z-direction extends parallel to a basic-position beam axis (120),
- **characterized by** a control device (500) for controlling the assembly, into which a scanning pattern is coded that is realizable by the scanning movement along a scan line (5),
wherein the scanning movement has at least one lateral basic component (13) in the x-direction and/or y-direction, which is overlaid by components of synchronous direction changing movements in the z-direction and in the x-direction and/or y-direction, wherein the synchronous direction changing movements are synchronized with respect to one another such that, in a positive lateral basic component (13) of the scanning movement in the x-direction and/or y-direction, the direction changing movements in the x-direction and/or y-direction run opposite to the direction changing movements in the z-direction, and that, in a negative lateral basic component (13) of the scanning movement in the x-direction and/or y-direction, the direction changing movements in the x-direction and/or y-direction run in the same direction as the direction changing movements in the z-direction.

16. Ophthalmological therapy assembly according to Claim 15, **characterized by** a scanning pattern having mutually adjacent strokes (7) having inclination angles (α) with respect to the beam axis (12), wherein a stroke (7) comprises an aligned part of the scan line (5) and is realized by a connected series of focus effective regions (8) of the focused radiation (1), wherein the inclination angles (α) of the strokes are greater than or equal to the focusing angle (φ) of the focused radiation (1).

17. Control program product configured for coding a control device (500) of an ophthalmological therapy assembly for processing a surface (11) in a processing volume (300) of a transparent material (3) of an eye by means of focused radiation (1) according to one of Claims 1 to 16, which contains
- a device for generating (100) radiation (1),
- an optical unit (2, 200, 201, 202) for focusing the radiation (1) in a focus (4) in the processing volume (300) that is describable with three spatial directions x, y and z, wherein the focus (4) of the focused radiation comprises a focusing angle (φ) and the focused radiation comprises a beam axis (12), and
- a device for changing the position (400) of the focus (4) in the processing volume (300) that is describable with three spatial directions x, y and z, wherein the z-direction extends parallel to a basic-position beam axis (120) of the focused radiation (12),
wherein the control device is coded
- such that the position of the focus (4) is changed by a slow scanning movement in every desired direction determined by the three spatial directions in the processing volume (300) of the transparent material (3) and a fast scanning movement, independent of the slow scanning movement, in a partial region (600) of the processing volume (300) in every desired direction determined by the three spatial directions, and wherein the partial region (600) of the fast scanning movement is moved owing to the slow scanning movement in the entire processing volume (300), or
- that the position of the focus (4) of the focused radiation is changed by a scanning movement in the processing volume (300) of the transparent material (3) in every desired direction determined by the three spatial directions x, y and z, and a scanning pattern is generated by the scanning movement in the transparent material (3) such that a scanning movement with a lateral basic component (13) in the x-direction and/or in the y-direction is overlaid by components of synchronous direction changing movements in the z-direction and in the x-direction and/or y-direction, wherein the direction changing movements are synchronized with respect to one another such that, in a positive lateral basic component (13) of the scanning movement in the x-direction and/or y-direction, the direction changing movements in the x-direction and/or y-direction run opposite to the direction changing movements in the z-direction, and that, in a negative lateral basic component (13) of the scanning movement in the x-direction and/or y-direction, the direction changing movements in the x-direction and/or y-direction run in the same direction as the direction changing movements in the z-direction.

18. Planning unit, which is contained in a control device (500) of an ophthalmological therapy assembly for processing a surface (11) in a processing volume (300) of a transparent material (3) of an eye by means of focused radiation (1), or which is configured, as an independent unit of the control device (500), to communicate with other units of the control device (500) via communication paths (501),
- wherein the ophthalmological therapy assembly furthermore contains
- a device for generating (100) radiation (1),
- an optical unit (2, 200, 201, 202) for focusing the radiation (1) in a focus (4) in the processing volume (300) that is describable with three spatial directions x, y and z, wherein the focus (4) of the focused radiation comprises a focusing angle (φ) and the focused radiation comprises a beam axis (12) and
- a device for changing the position (400) of the focus (4) in the processing volume (300) that is describable with three spatial directions x, y and z, wherein the z-direction extends parallel to a basic-position beam axis (120) of the focused radiation (12),
wherein the planning unit is configured to provide all the steps of therapy management for performing a method for processing a surface (11) in a processing volume (300) of a transparent material (3) of an eye by way of focused radiation (1) in a manner such
- that the position of the focus (4) is changed by a slow scanning movement in every desired direction determined by the three spatial directions in the processing volume (300) of the transparent material (3) and a fast scanning movement, independent of the slow scanning movement, in a partial region (600) of the processing volume (300) in every desired direction determined by the three spatial directions, and wherein the partial region (600) of the fast scanning movement is moved owing to the slow scanning movement in the entire processing volume (300), or
- that the position of the focus (4) of the focused radiation is changed by a scanning movement in the processing volume (300) of the transparent material (3) in every desired direction determined by the three spatial directions x, y and z, and a scanning pattern is generated by the scanning movement in the transparent material (3) such that a scanning movement with a lateral basic component (13) in the x-direction and/or in the y-direction is overlaid by components of synchronous direction changing movements in the z-direction and in the x-direction and/or y-direction, wherein the direction changing movements are synchronized with respect to one another such that, in a positive lateral basic component (13) of the scanning movement in the x-direction and/or y-direction, the direction changing movements in the x-direction and/or y-direction run opposite to the direction changing movements in the z-direction, and that, in a negative lateral basic component (13) of the scanning movement in the x-direction and/or y-direction, the direction changing movements in the x-direction and/or y-direction run in the same direction as the direction changing movements in the z-direction.

19. Planning unit according to Claim 18, containing a selection table of scanning patterns and/or an algorithm for creating a scanning pattern.

## Revendications

1. Ensemble de thérapie ophtalmologique destiné à traiter une surface (11) dans un volume de traitement (300) d'une matière transparente (3) d'un œil au moyen d'un rayonnement focalisé (1), ledit ensemble comprenant
- un dispositif de génération (100) d'un rayonnement (1),
- un système optique destiné à focaliser (2, 200, 202) le rayonnement (1) à un foyer (4) dans le volume de traitement (300), le foyer (4) du rayonnement focalisé ayant un angle de focalisation (φ) et le rayonnement focalisé comportant un axe de faisceau (12),
- un dispositif de modification de position (400) du foyer (4) dans le volume de traitement (300) qui peut être décrit avec trois directions spatiales x, y et z, la direction z s'étendant parallèlement à un axe de faisceau de position de base (120) du rayonnement focalisé (12),
- un dispositif de commande (500) destiné à commander l'ensemble de thérapie ophtalmologique,
- **caractérisé en ce que** le dispositif de modification de la position (400) du foyer (4) est conçu pour effectuer, dans une direction quelconque déterminée par les trois directions spatiales, un mouvement de balayage lent dans le volume de traitement (300) de la matière transparente (3) et pour effectuer, dans une direction quelconque déterminée par les trois directions spatiales, un mouvement de balayage rapide indépendant du mouvement de balayage lent dans une sous-zone (600) du volume de traitement (300),
- la sous-zone (600) du mouvement de balayage rapide pouvant être déplacée par le mouvement de balayage lent dans tout le volume de traitement (300).

2. Ensemble de thérapie ophtalmologique selon la revendication 1, **caractérisé par** un dispositif de génération (100) d'un rayonnement (1) qui comprend un laser.

3. Ensemble de thérapie ophtalmologique selon la revendication 2, le laser étant un laser pulsé, en particulier un laser à impulsions courtes, de préférence un laser femtoseconde.

4. Ensemble de thérapie ophtalmologique selon l'une des revendications 1 à 3, un motif de balayage étant codé dans le dispositif de commande (500), lequel motif comporte une séquence de zones focales effectives (8, 81, 82) du rayonnement focalisé (1) le long d'une ligne de balayage (5) dans le volume de traitement (300) de sorte que les zones focales effectives (8, 81) déjà réalisées soient toujours disposées, pour les zones focales effectives (8, 82) qui restent à réaliser, à l'extérieur d'un cône de foyer (6) qui est formé par le foyer (4) du rayonnement focalisé (1) et l'angle de focalisation (φ).

5. Ensemble de thérapie ophtalmologique selon l'une des revendications 1 à 4, **caractérisé par** un dispositif de modification de position (400) du foyer (4), lequel dispositif comporte un scanner z rapide (401) et au moins un scanner latéral rapide (402, 403) destinés à effectuer le mouvement de balayage rapide dans la sous-zone (600) du volume de traitement (300) en plus d'un scanner z lent (411) et d'au moins un scanner latéral lent (412, 413) destinés à effectuer le mouvement de balayage lent dans le volume de traitement (300).

6. Ensemble de thérapie ophtalmologique selon la revendication 5, **caractérisé par** un scanner latéral rapide (402, 403), qui est formé par un scanner x rapide (402) et qui est présent en plus d'un autre scanner latéral rapide qui est formé par un scanner y rapide (403), ou par un scanner latéral rapide (402, 403) qui est formé par un scanner R rapide dont le mouvement de balayage peut être orienté dans un plan x-y perpendiculairement à l'axe de faisceau de position de base (120) par rotation sur un axe de rotation parallèle à l'axe de faisceau de position de base (120).

7. Ensemble de thérapie ophtalmologique selon la revendication 5 ou 6, **caractérisé par** un scanner z rapide, qui comprend un élément formant lentille oscillant dans la direction z, un scanner latéral rapide qui comprend un élément formant miroir x-y mobile sur deux axes ou deux éléments formant miroirs qui sont mobiles chacun sur un axe et dont les axes sont de préférence perpendiculaires l'un à l'autre, un scanner z lent qui comprend un élément formant lentille qui peut être mû au choix dans la direction z, et un scanner latéral lent qui comprend une optique de focalisation qui peut être déplacé au choix dans le plan latéral.

8. Ensemble de thérapie ophtalmologique selon l'une des revendications 5 à 7, conçu pour effectuer un mouvement de balayage rapide par changement de direction synchrone d'au moins deux scanners rapides (401, 402, 403), notamment par des mouvements oscillatoires synchrones d'au moins deux scanners rapides (401, 402, 403).

9. Ensemble de thérapie ophtalmologique selon la revendication 8, **caractérisé en ce que** l'un des au moins deux scanners rapides (401, 402, 403) est un scanner résonant à oscillation libre, et tous les autres des au moins deux scanners rapides (401, 402, 403) sont synchronisés sur le scanner résonant.

10. Ensemble de thérapie ophtalmologique selon la revendication 8 ou 9, **caractérisé par** des mouvements oscillatoires synchrones du scanner z rapide (401) et de l'au moins un scanner latéral rapide (402, 403).

11. Ensemble de thérapie ophtalmologique selon la revendication 10, le mouvement de balayage lent comportant une composante latérale (13) dans la direction x et/ou dans la direction y et les mouvements oscillatoires du scanner z rapide (401) et de l'au moins un scanner latéral rapide (402, 403) étant synchronisés de telle sorte que
- dans le cas où le mouvement de balayage lent dans la direction x et/ou y comporte une composante latérale positive (13), les mouvements oscillatoires du scanner latéral rapide, notamment du scanner x rapide et/ou du scanner y rapide, soient en opposition de phase avec le mouvement oscillatoire du scanner z rapide, et
- dans le cas où le mouvement de balayage lent dans la direction x et/ou y comporte une composante latérale négative (13), les mouvements oscillatoires du scanner latéral rapide, notamment du scanner x rapide et/ou du scanner y rapide, soient en phase avec le mouvement oscillatoire du scanner z rapide.

12. Ensemble de thérapie ophtalmologique selon l'une des revendications 4 à 11, **caractérisé par** un dispositif de commande (500) dans lequel un motif de balayage est codé qui comporte des traits (7) mutuellement adjacents avec des angles d'inclinaison (α) par rapport à l'axe de faisceau (12), un trait (7) comprenant une partie unidirectionnelle d'une ligne de balayage (5) et étant réalisé par une succession de zones focales effectives (8) du rayonnement focalisé (1), et les angles d'inclinaison (α) des traits (7) par rapport à l'axe de faisceau (12) étant toujours supérieurs ou égaux à l'angle de focalisation (φ) du rayonnement focalisé (1).

13. Ensemble de thérapie ophtalmologique selon la revendication 12, **caractérisé par** un codage du dispositif de commande (500) de sorte que la formation des traits (7) par succession de zones focales effectives (8) du rayonnement focalisé (1) est réalisée toujours dans un mouvement ascendant (14) ou toujours dans un mouvement descendant (15) ou alternativement dans un mouvement ascendant (13) et dans un mouvement descendant (14) le long de la ligne de balayage (5).

14. Ensemble de thérapie ophtalmologique selon l'une des revendications 1 à 13, le dispositif de génération d'un rayonnement comprenant un laser pulsé ayant un taux de répétition d'impulsion laser (f_{L}) et une distance (9) d'une zone de focale effective (8) à une zone de focale effective (8) précédente étant déterminée par le taux de répétition d'impulsion laser (f_{L}) et une vitesse de balayage totale qui résulte des vitesses de balayage des mouvements de balayage lents et rapides, **caractérisé par** un dispositif de commande (500) qui est conçu pour supprimer une impulsion laser lorsque sa zone de focale effective (8) devient inférieure à une distance minimale (9) par rapport à la zone focale effective (8) précédente.

15. Ensemble de thérapie ophtalmologique destiné à traiter une surface (11) dans un volume de traitement (300) d'une matière transparente (3) d'un œil au moyen d'un rayonnement focalisé (1), ledit ensemble comprenant
- un dispositif de génération (100) d'un rayonnement (1),
- un système optique (2, 200, 201, 202) destiné à focaliser le rayonnement (1) dans un foyer (4) dans le volume de traitement (300), le foyer (4) du rayonnement focalisé ayant un angle de focalisation (φ) et le rayonnement focalisé comportant un axe de faisceau (12),
- un dispositif de modification de la position (400) du foyer (4) au moyen d'un mouvement de balayage dans une direction variable quelconque dans le volume de traitement (300) qui peut être décrit avec trois directions spatiales x, y et z, la direction z s'étendant parallèlement à un axe de faisceau de position de base (120),
- **caractérisé par** un dispositif de commande (500) destiné à commander l'ensemble dans lequel un motif de balayage est codé qui peut être réalisé par le mouvement de balayage le long d'une ligne de balayage (5),
le mouvement de balayage comportant au moins une composante de base latérale (13) dans la direction x et/ou y, qui est superposée à des composantes de mouvements de changement de direction synchrones dans la direction z et dans la direction x et/ou la direction y, les mouvements de changement de direction synchrones étant synchronisés entre eux de telle sorte que, lorsque le mouvement de balayage dans la direction x et/ou y comporte une composante de base latérale positive (13), les mouvements de changement de direction dans la direction x et/ou y soient de sens opposés aux mouvements de changement de direction dans la direction z et, lorsque le mouvement de balayage dans la direction x et/ou y comporte une composante de base latérale négative (13), les mouvements de changement de direction dans la direction x et/ou y soient de même sens que les mouvements de changement de direction dans la direction z.

16. Ensemble de thérapie ophtalmologique selon la revendication 15, **caractérisé par** un motif de balayage qui comporte des traits mutuellement adjacents (7) ayant des angles d'inclinaison (α) par rapport à l'axe du faisceau (12), un trait (7) comprenant une partie unidirectionnelle de la ligne de balayage (5) et étant réalisé par une succession de zones focales effectives (8) du rayonnement focalisé (1), l'angle d'inclinaison (α) du trait étant supérieur ou égal à l'angle de focalisation (φ) du rayonnement focalisé (1).

17. Progiciel conçu pour coder un dispositif de commande (500) d'un ensemble de thérapie ophtalmologique destiné à traiter une surface (11) dans un volume de traitement (300) d'une matière transparente (3) d'un œil au moyen d'un rayonnement focalisé (1) selon l'une des revendications 1 à 16, lequel ensemble comporte
- un dispositif de génération (100) d'un rayonnement (1),
- un système optique (2, 200, 201, 202) destiné à focaliser le rayonnement (1) à un foyer (4) du volume de traitement (300) qui peut s'écrire avec trois directions spatiales x, y et z, le foyer (4) du rayonnement focalisé ayant un angle de focalisation (φ) et le rayonnement focalisé comportant un axe de faisceau (12), et
- un dispositif de changement de position (400) du foyer (4) dans le volume de traitement (300), qui peut être décrit avec trois directions spatiales x, y et z, la direction z s'étendant parallèlement à un axe de faisceau de position de base (120) du rayonnement focalisé (12),
le dispositif de commande étant codé de sorte que
- la position du foyer (4) soit modifiée par un mouvement de balayage lent dans une direction quelconque déterminée par les trois directions spatiales dans le volume de traitement (300) de la matière transparente (3) et un mouvement de balayage rapide indépendant du mouvement de balayage lent dans une sous-zone (600) du volume de traitement (300) dans une direction quelconque déterminée par les trois directions spatiales, et la sous-zone (600) du mouvement de balayage rapide étant déplacée par le mouvement de balayage lent dans tout le volume de traitement (300), ou
- la position du foyer (4) du rayonnement focalisé soit modifiée par un mouvement de balayage dans le volume de traitement (300) de la matière transparente (3) dans une direction quelconque déterminée par les trois directions spatiales x, y et z, et un motif de balayage soit généré par le mouvement de balayage dans la matière transparente (3) de telle sorte qu'un mouvement de balayage ayant une composante de base latérale (13) dans la direction x et/ou dans la direction y soit superposé à composantes de mouvements de changement de direction synchrones dans la direction z et dans la direction x et/ou la direction y, les mouvements de changement de direction étant synchronisés entre eux de telle sorte que, lorsque le mouvement de balayage dans la direction x et/ou y comporte une composante de base latérale positive (13), les mouvements de changement de direction dans la direction x et/ou y soient effectués dans un sens opposé à celui des mouvements de changement de direction dans la direction z et, lorsque le mouvement de balayage dans la direction x et/ou y comporte une composante de base latérale négative (13), les mouvements de changement de direction dans la direction x et/ou y soient effectués dans le même sens que les mouvements de changement de direction dans la direction z.

18. Unité de planification qui est contenue dans un dispositif de commande (500) d'un ensemble de thérapie ophtalmologique destiné à traiter une surface (11) dans un volume de traitement (300) d'une matière transparente (3) d'un œil au moyen d'un rayonnement focalisé (1) ou qui est conçue en tant qu'unité indépendante du dispositif de commande (500) pour être en liaison avec d'autres unités du dispositif de commande (500) par le biais de voies de communication (501),
- l'ensemble de thérapie ophtalmologique comportant en outre
- un dispositif de génération (100) de rayonnement (1)
- un système optique (2, 200, 201, 202) de focalisation du rayonnement (1) à un foyer (4) dans le volume de traitement (300) qui peut être décrit par trois directions spatiales x, y et z, le foyer (4) du rayonnement focalisé ayant un angle de focalisation (φ) et le rayonnement focalisé comportant un axe de faisceau (12) et
- a un dispositif de changement de position (400) du foyer (4) dans le volume de traitement (300), qui peut être décrit avec trois directions spatiales x, y et z, la direction z s'étendant parallèlement à un axe de faisceau de position de base (120) du rayonnement focalisé (12),
l'unité de planification étant conçue pour prévoir toutes les étapes d'une gestion de thérapie destinées à la mise en œuvre d'un procédé de traitement d'une surface (11) dans un volume de traitement (300) d'une matière transparente (3) d'un œil au moyen d'un rayonnement (1) de sorte que
- la position du foyer (4) soit modifiée par un mouvement de balayage lent dans une direction quelconque déterminée par les trois directions spatiales dans le volume de traitement (300) de la matière transparente (3) et un mouvement de balayage rapide indépendant du mouvement de balayage lent dans une sous-zone (600) du volume de traitement (300) dans une direction quelconque déterminée par les trois directions spatiales, et la sous-zone (600) du mouvement de balayage rapide étant déplacée par le mouvement de balayage lent dans tout le volume de traitement (300), ou
- la position du foyer (4) du rayonnement focalisé soit modifiée par un mouvement de balayage dans le volume de traitement (300) de la matière transparente (3) dans une direction quelconque déterminée par les trois directions spatiales x, y et z, et un motif de balayage soit généré par le mouvement de balayage dans la matière transparente (3) de telle sorte qu'un mouvement de balayage ayant une composante de base latérale (13) dans la direction x et/ou dans la direction y soit superposé à des composantes de mouvements de changement de direction synchrones dans la direction z et dans la direction x et/ou la direction y, les mouvements de changement de direction étant synchronisés entre eux de telle sorte que, lorsque le mouvement de balayage dans la direction x et/ou y comporte une composante de base latérale positive (13), les mouvements de changement de direction dans la direction x et/ou y soient effectués dans un sens opposé à celui des mouvements de changement de direction dans la direction z et, lorsque le mouvement de balayage dans la direction x et/ou y comporte une composante de base latérale négative (13), les mouvements de changement de direction dans la direction x et/ou y soient effectués dans le même sens que les mouvements de changement de direction dans la direction z.

19. Unité de planification selon la revendication 18, qui contient une table de sélection de motifs de balayage et/ou un algorithme de création d'un motif de balayage.
